# EUROPEAN PATENT SPECIFICATION

(11) **EP 3 330 751 B1**
(45) Date of publication and mention of the grant of the patent: **26.05.2021**
(21) Application number: 16841986.9
(22) Date of filing: 26.08.2016
(51) Int. Cl.: G02B 5/23, G02C 7/10, C08G 83/00, C09D 201/00, C09D 175/00, C09D 171/00

(54) **METHOD OF PRODUCING A PHOTOCHROMIC LAMINATE**
VERFAHREN ZUR HERSTELLUNG EINES PHOTOCHROMEN LAMINAT
PROCÉDÉ DE FABRICATION D'UN LAMINÂT PHOTOCHROMIQUE

(30) Priority: 03.09.2015 JP 2015173953
(43) Date of publication of application: 06.06.2018
(73) Proprietor: Tokuyama Corporation, Shunan-shi, Yamaguchi 745-0053 (JP)
(72) Inventor: MORI, Katsuhiro, Shunan-shi Yamaguchi 745-0053 (JP); SHIMIZU, Yasutomo, Shunan-shi Yamaguchi 745-0053 (JP); MOMODA, Junji, Shunan-shi Yamaguchi 745-0053 (JP)
(74) Representative: Beckmann, Claus
(86) International application number: PCT/JP2016/075739
(87) International publication number: WO 2017/038957

(56) References cited:
- WO-A1-01/83566
- WO-A1-2012/141250
- WO-A1-2012/144460
- WO-A1-2015/068798
- WO-A1-2015/068798
- WO-A1-2016/143910
- JP-A- 2009 204 727
- JP-A- 2010 138 258

## Description

### TECHNICAL FIELD

The present invention relates to a method of producing a laminate having a photochromic layer obtained from a novel photochromic coating composition.

### BACKGROUND ART

Photochromic compounds typified by chromene compounds, fulgide compounds and spirooxazine compounds have a characteristic feature (photochromic properties) that they change their colors swiftly upon exposure to light including ultraviolet light such as sunlight or light from a mercury lamp and return to their original colors when they are put in the dark by stopping their exposure to light and are used for various purposes, especially optical materials, making use of this characteristic feature.

For example, photochromism is also applied in the field of spectacle lenses. Photochromic spectacle lenses which are obtained by using a photochromic compound function as sunglasses which are quickly colored outdoors where they are irradiated with light including ultraviolet light such as sunlight and as ordinary transparent eyeglasses which are faded indoors where there is no irradiation, and demand for the photochromic spectacle lenses is growing nowadays.

As for photochromic spectacle lenses, plastic lenses are preferred from the viewpoints of lightweight and safety. Photochromic properties are generally provided to the plastic lenses by compounding the above photochromic compounds. As the method of producing a photochromic spectacle lens, there are known a method in which a photochromic compound is dissolved in a monomer and the monomer is polymerized to obtain a photochromic lens directly (to be referred to as "kneading method" hereinafter) and a method in which a layer having photochromic properties (to be also referred to as "photochromic layer" hereinafter) is formed on the surface of a plastic having no photochromic properties (to be referred to as "lamination method" hereinafter). Various technologies have been proposed for the lamination method (refer to WO2011/125956 and WO2003/011967) since this method can provide photochromic properties to plastic lenses having various refractive indices.

As for these photochromic compounds and plastic optical articles having photochromic properties and comprising these photochromic compounds, the following photochromic properties are especially important in all of these technologies: (I) the degree of coloration upon exposure to ultraviolet light (to be referred to as "color optical density" hereinafter) should be high and (II) the speed from the stoppage of the application of ultraviolet light to the time when the optical article returns to its original state (to be referred to as "fading speed" hereinafter) should be high. Further, moldability at the time of producing a photochromic spectacle lens and the surface hardness of the photochromic layer are also important.

With these technologies as the background, photochromic plastic lenses (optical materials) having high color optical density and high fading speed are proposed. The development of photochromic compositions comprising various polymerizable monomers and photochromic compounds (especially chromene compounds) is under way in the lamination method.

WO2011/125956 and WO2003/011967 disclose a method in which a photochromic composition comprising a specific (meth)acrylic polymerizable monomer and a photochromic compound is applied to a plastic lens by spin coating and optically cured (to be also referred to as "coating method" hereinafter) and a method in which a photochromic composition comprising a specific (meth)acrylic polymerizable monomer and a photochromic compound is poured into a space between a plastic lens and a glass mold held by an elastomer gasket, adhesive tape or spacer and polymerized and cured (to be also referred to as "two-stage polymerization method" hereinafter). WO2001/055269 discloses a coating method in which a photochromic composition comprising a polyurethane monomer including a specific isocyanate compound and a polyol and a photochromic compound is applied to a plastic lens by spin coating and thermally cured. Laminates (photochromic plastic lenses) having excellent photochromic properties can be manufactured by using these photochromic compositions.

Further, WO2015/068798 discloses a coating composition comprising a polyrotaxane as a special polymer and monomer. More specifically, a coating composition comprising a polyrotaxane, a (meth)acrylic polymerizable monomer and a photochromic compound is disclosed in this WO2015/068798.

However, due to growing demand for the improvement of the performance of a photochromic plastic lens, a photochromic coating composition which can provide a higher performance lens with high moldability at a high yield has been desired. Further, a photochromic composition which can develop excellent surface hardness has been desired from the viewpoint of preventing the surface of the obtained photochromic plastic lens from being scratched.

### DISCLOSURE OF THE INVENTION

It is therefore an object of the present invention to provide a method of producing a photochromic laminate having a photochromic layer obtained from a photochromic coating composition which can provide a photochromic plastic lens having excellent photochromic properties such as color optical density and fading speed with high moldability at a high yield and is excellent in the surface hardness of the obtained photochromic plastic lens.

Other objects and advantages of the present invention will become apparent from the following description.

The inventors of the present invention conducted intensive studies to attain the above object. As a result, they succeeded in attaining the above object by combining a photochromic compound with a polyrotaxane and a polyurethane resin and/or precursor thereof.

That is, according to the present invention, there is provided a photochromic coating composition which comprises (A) a polyrotaxane having a composite molecular structure formed by an axial molecule and a plurality of cyclic molecules clathrating the axial molecule, (B) a photochromic compound and (C) a polyurethane resin precursor (C2) thereof.

In the present invention, the above polyrotaxane (A) is a molecular complex having a structure that a chain axial molecule passes through the inside of each of the rings of a plurality of cyclic molecules, a bulky group is bonded to both ends of the axial molecule, and the cyclic molecules cannot be removed from the axial molecule due to steric hindrance.

The molecular complex like the polyrotaxane is called "supramolecule".

The polyrotaxane (A) in the present invention can take the following preferred modes.
(1) A side chain having at least one polymerizable functional group selected from OH group, SH group, NH₂ group, NCO group and NCS group is introduced into at least one of the ring contained in each of the cyclic molecules of the polyrotaxane (A).
(2) The polyurethane resin precursor (C2) includes (C2-1) a polyiso(thio)cyanate compound having at least two isocyanate groups and/or isothiocyanate groups in one molecule and (C2-2) a poly(thi)ol compound having at least two hydroxyl groups and/or thiol groups in one molecule.
(3) The polyurethane resin precursor (C2) further includes (C2-3) a mono(thi)ol compound having one hydroxyl group or thiol group in one molecule.

According to the present invention, there is provided a laminate having a photochromic layer obtained from the photochromic coating composition of the present invention on an optical substrate such as a plastic lens (to be referred to photochromic laminate hereinafter).

### BRIEF DESCRIPTION OF THE DRAWINGS

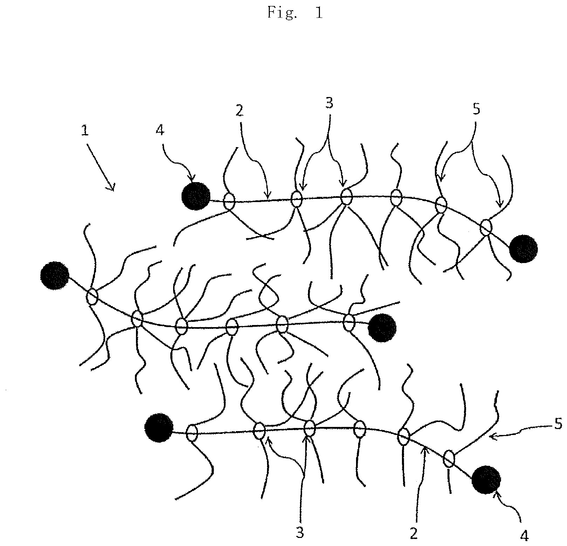

Fig. 1 is a schematic diagram showing the molecular structure of a polyrotaxane used in the present invention.

### MODE FOR CARRYING OUT THE INVENTION

The photochromic coating composition is a coating agent which generally comprises a photochromic compound, a polymerizable monomer and/or a polymer resin and is used to provide photochromic properties to an optical substrate such as a plastic lens by a technique such as spin coating.

The photochromic coating composition of the present invention comprises (A) a polyrotaxane, (B) a photochromic compound and (C) a polyurethane resin and/or precursor thereof and may further comprise other known compounding agents.

### (A)Polyrotaxane;

The polyrotaxane is a known compound, and the polyrotaxane molecule represented by "1" as a whole has a composite molecular structure formed by a chain axial molecule "2" and cyclic molecules "3" as shown in Fig. 1. That is, a plurality of the cyclic molecules "3" clathrate the chain axial molecule "2", and the axial molecule "2" passes through the inside of each of the rings of the cyclic molecules "3". Therefore, the cyclic molecules "3" can freely slide over the axial molecule "2" but a bulky terminal group "4" is formed at both ends of the axial molecule "2" to prevent the cyclic molecules "3" from falling off from the axial molecule "2". "5" denotes the side chains of the cyclic molecules "3".

Since the cyclic molecules "3" can slide over the axial molecule "2" as described above, a space which can allow for the reversible reaction of the photochromic compound is secured, thereby making it possible to obtain high color optical density and high fading speed and to mitigate stress caused by polymerization shrinkage which occurs at the time of forming the photochromic layer. Therefore, high moldability is obtained without producing an appearance defect.

In the above polyrotaxane, various axial molecules are known. For example, the chain part may be linear or branched as long as the axial molecule can pass through the rings of the cyclic molecules and is generally formed from a polymer.

Examples of the polymer forming the chain part of the axial molecule include polyvinyl alcohol, polyvinyl pyrrolidone, cellulose-based resins (such as carboxymethyl cellulose, hydroxyethyl cellulose and hydroxypropyl cellulose), polyacrylamide, polyethylene oxide, polyethylene glycol, polypropylene glycol, polyvinyl acetal, polyvinyl methyl ether, polyamine, polyethylene imine, casein, gelatin, starch, olefin-based resins (such as polyethylene and polypropylene), polyester, polyvinyl chloride, styrene-based resins (such as polystyrene and acrylonitrile-styrene copolymer resin), acrylic resins (such as poly(meth)acrylic acid, polymethyl methacrylate, polymethyl acrylate and acrylonitrile-methyl acrylate copolymer resin), polycarbonate, polyurethane, vinyl chloride-vinyl acetate copolymer resin, polyvinyl butyral, polyisobutylene, polytetrahydrofuran, polyaniline, acrylonitrile-butadiene-styrene copolymer or ABS resin, polyamides (such as nylon), polyimide, polydienes (such as polyisoprene and polybutadiene), polysiloxanes (such as polydimethylsiloxane), polysulfone, polyimine, polyacetic anhydride, polyurea, polysulfide, polyphosphazene, polyketone polyphenylene and polyhalo olefins. These polymers may be copolymerized or modified.

In the present invention, the polymer forming the chain part is particularly preferably polyethylene glycol, polyisoprene, polyisobutylene, polybutadiene, polypropylene glycol, polytetrahydrofuran, polydimethylsiloxane, polyethylene, polypropylene, polyvinyl alcohol or polyvinyl methyl ether and polyethylene glycol.

Further, the bulky group formed at the both ends of the chain part is selected from adamantyl group, trityl group, fluoresceinyl group, dinitrophenyl group and pyrenyl group. Adamantyl group is preferred from the viewpoint of introduction ease.

Although the molecular weight of the above-described axial molecule is not particularly limited, when it is too high, its compatibility with another component, for example, the polymerizable monomer (C) which is suitably blended tends to lower and when it is too low, the mobility of the cycle molecules becomes low, whereby photochromic properties tend to deteriorate. From this point of view, the weight average molecular weight Mw of the axial molecule is preferably 1,000 to 100,000, more preferably 5,000 to 80,000, particularly preferably 10,000 to 50,000.

Each of the cyclic molecules should have a ring large enough to clathrate the above axial molecule. Examples of this ring include cyclodextrin ring, crown ether ring, benzo-crown ring, dibenzo-crown ring and dicyclohexano-crown ring, out of which cyclodextrin ring is particularly preferred.

The cyclodextrin ring has α-form (ring inner diameter of 0.45 to 0.6 nm), β-form (ring inner diameter of 0.6 to 0.8 nm) or γ-form (ring inner diameter of 0.8 to 0.95 nm) . In the present invention, α-cyclodextrin ring and γ-cyclodextrin ring are preferred, and α-cyclodextrin ring is most preferred.

A plurality of the cyclic molecules having the above ring clathrate one axial molecule. In general, when the maximum number of cyclic molecules capable of clathrating one axial molecule is 1, the number of clathrating cyclic molecules is preferably 0.001 to 0.6, more preferably 0.002 to 0.5, much more preferably 0.003 to 0.4. When the number of clathrating cyclic molecules is too large, the cyclic molecules are densely existent for one axial molecule, whereby the mobility of the cyclic molecules becomes low and photochromic properties and moldability tend to deteriorate. When the number of clathrating cyclic molecules is too small, the space between adjacent axial molecules becomes narrow and the space which can allow for the reversible reaction of the photochromic compound molecule becomes small, whereby photochromic properties and moldability tend to deteriorate as well.

The maximum number of cyclic molecules clathrating one axial molecule can be calculated from the length of the axial molecule and the thickness of each of the rings of the cyclic molecules.

For example, when the chain part of the axial molecule is formed from polyethylene glycol and the ring of the cyclic molecule is an α-cyclodextrin ring, the maximum number of clathrating cyclic molecules is calculated as follows.

That is, two recurring units [-CH₂-CH₂O-] of polyethylene glycol approximate the thickness of one α-cyclodextrin ring. Therefore, the number of the recurring units is calculated from the molecular weight of polyethylene glycol to obtain 1/2 of the number of the recurring units as the maximum number of clathrating cyclic molecules. Based on the condition that the maximum number of clathrating cyclic molecules is 1.0, the number of clathrating cyclic molecules is adjusted to the above range.

Further, in the present invention, side chains may be introduced into the ring of the above-described cyclic molecule. The side chains are represented by "5" in Fig.1.

That is, by introducing the side chains "5" into the ring, an appropriate space can be surely formed between adjacent axial molecules, thereby making it possible to secure a space which can allow for the reversible reaction of the photochromic compound molecule and to develop excellent photochromic properties.

The above side chain is preferably formed from repetitions of an organic chain having 3 to 20 carbon atoms and has an average weight molecular weight of 300 to 10,000, preferably 350 to 8,000, more preferably 350 to 5,000, most preferably 400 to 1,500. When the side chain is too small, its function of securing the space capable of allowing for the reversible reaction of the photochromic compound molecule becomes unsatisfactory and when the side chain is too large, it is difficult to closely mix the photochromic compound which will be described hereinafter with the polyrotaxane, thereby making it difficult to make full use of the space secured by the polyrotaxane.

Further, the above side chain is introduced by modifying the functional groups of the ring of each cyclic molecule. For example, the α-cyclodextrin ring has 18 hydroxyl groups as the functional groups through which the side chain is introduced. That is, a maximum of 18 side chains can be introduced into one α-cyclodextrin ring. In the present invention, to fully obtain the function of the above-described side chain, not less than 6 %, particularly not less than 30 % of the total number of all the functional groups of the ring are preferably modified by the side chain. When the side chain is bonded to 9 out of the 18 hydroxyl groups of the above α-cyclodextrin ring, the degree of modification is 50 %.

In the present invention, the above side chain (organic chain) may be linear or branched as long as its size falls within the above range. A side chain having an appropriate size can be introduced by reacting a suitable compound with the functional groups of the above ring by making use of ring-opening polymerization, radical polymerization, cationic polymerization, anionic polymerization, RAFT polymerization or NMP polymerization.

For example, a side chain derived from a cyclic compound such as a cyclic lactone, cyclic ether, cyclic acetal, cyclic amine, cyclic carbonate, cyclic iminoether or cyclic thiocarbonate can be introduced by ring-opening polymerization. From the viewpoints of acquisition ease, high reactivity and easy control of size (molecular weight), a cyclic ether, cyclic siloxane, lactone or cyclic carbonate is preferably used. Preferred examples of the cyclic compound are given below.
Cyclic ethers; ethylene oxide, 1,2-propylene oxide, epichlorohydrin, epibromohydrin, 1,2-butylene oxide, 2,3-butylene oxide, isobutylene oxide, oxetane, 3-methyloxetane, 3,3-dimethyloxetane, tetrahydrofuran, 2-methyl tetrahydrofuran and 3-methyl tetrahydrofuran
Cyclic siloxanes; hexamethyl cyclotrisiloxane and octamethyl cyclotetrasiloxane
Lactones;
4-membered cyclic lactones such as β-propiolactone, β-methyl propiolactone and L-serine-β-lactone, and the like.
5-membered cyclic lactones such as γ-butyrolactone, γ-hexanolactone, γ-heptanolactone, γ-octanolactone, γ-decanolactone, γ-dodecanolactone, α-hexyl-γ-butyrolactone, α-heptyl-γ-butyrolactone, α-hydroxy-γ-butyrolactone, γ-methyl-γ-decanolactone, α-methylene-γ-butyrolactone, α,α-dimethyl-γ-butyrolactone, D-erythronolactone, α-methyl-γ-butyrolactone, γ-nonanolactone, DL-pantolactone, γ-phenyl-γ-butyrolactone, γ-undecanolactone, γ-valerolactone, 2,2-pentamethylene-1,3-dioxolan-4-one, α-bromo-y-butyrolactone, γ-crotonolactone, α-methylene-y-butyrolactone, α-methacryloyloxy-γ-butyrolactone and β-methacryloyloxy-γ-butyrolactone, and the like.

6-membered cyclic lactones such as δ-valerolactone, δ-hexanolactone, δ-octanolactone, δ-nonanolactone, δ-decanolactone, δ-undecanolactone, δ-dodecanolactone, δ-tridecanolactone, δ-tetradecanolactone, DL-mevalonolactone, 4-hydroxy-1-cyclohexane carboxylic acid δ-lactone, monomethyl-δ-valerolactone, monoethyl-δ-valerolactone, monohexyl-δ-valerolactone, 1,4-dioxan-2-one and 1,5-dioxepan-2-one, and the like.

7-membered cyclic lactones such as non-alkyl-ε-caprolactone, dialkyl-ε-caprolactone, monomethyl-ε-caprolactone monoethyl-ε-caprolactone, monohexyl-s-caprolactone, dimethyl-ε-caprolactone, di-n-propyl-ε-caprolactone, di-n-hexyl-ε-caprolactone, trimethyl-ε-caprolactone, triethyl-ε-caprolactone, tri-n-ε-caprolactone, ε-caprolactone, 5-nonyl-oxepan-2-one, 4,4,6-trimethyl-oxepan-2-one, 4,6,6-trimethyl-oxepan-2-one and 5-hydroxymethyl-oxepan-2-one, and the like.

8-membered cyclic lactones such as ξ-enantholactone, and the like.
other cyclic lactones such as lactone, lactide, dilactide, tetramethyl glycoside, 1,5-dioxepan-2-one and t-butyl caprolactone, and the like.

Cyclic carbonates such as ethylene carbonate, propylene carbonate, 1,2-butylene carbonate, glycerol 1,2-carbonate, 4-(methoxymethyl)-1,3-dioxolan-2-one, (chloromethyl)ethylene carbonate, vinylene carbonate, 4,5-dimethyl-1,3-dioxol-2-one, 4-chloromethyl-5-methyl-1,3-dioxol-2-one, 4-vinyl-1,3-dioxolan-2-one, 4,5-diphenyl-1,3-dioxolan-2-one, 4,4-dimethyl-5-methylene-1,3-dioxolan-2-one, 1,3-dioxan-2-one, 5-methyl-5-propyl-1,3-dioxolan-2-one and 5,5-diethyl-1,3-dioxolan-2-one

The above cyclic compounds may be used alone or even in combination of two or more.

In the present invention, lactones and cyclic carbonates are preferred, lactones such as ε-caprolactone, α-acetyl-γ-butyrolactone, α-methyl-γ-butyrolactone, γ-valerolactone and γ-butyrolactone are particularly preferred, and ε-caprolactone is most preferred.

When the side chain is to be introduced by reacting the cyclic compound through ring-opening polymerization, functional groups, for example, hydroxyl groups bonded to the ring have poor reactivity and therefore, it may be difficult to directly react a large molecule due to steric hindrance. In this case, to react, for example, caprolactone, there can be employed means for introducing the side chain through the ring-opening polymerization of caprolactone after a low-molecular weight compound such as propylene oxide is reacted with the functional group to carry out hydroxypropylation so as to introduce a highly reactive functional group (hydroxyl group).

Although the compound used to introduce the side chain by using radical polymerization is a radically polymerizable compound, each of the rings of the cyclic molecules of the polyrotaxane does not have an active site as a radical starting point. Therefore, prior to the reaction of the radically polymerizable compound, a compound for forming the radical starting point must be reacted with a functional group (hydroxyl group) of the ring to form the active site as the radical starting point.

A typical example of the compound for forming the radical starting point is an organic halogen compound exemplified by 2-bromoisobutyryl bromide, 2-bromobutyric acid, 2-bromopropionic acid, 2-chloropropionic acid, 2-bromoisobutyric acid, epichlorohydrin, epibromohydrin and 2-chloroethyl isocyanate.

That is, the organic halogen compound is bonded to the ring by a condensation reaction with a functional group of the ring of the cyclic molecule, thereby introducing a group containing a halogen atom, that is, an organic halogen compound residue.

A radical is produced in this organic halogen compound residue by the movement of a halogen atom upon radical polymerization to become the radical polymerization starting point from which radical polymerization proceeds.

The organic halogen compound residue which is a group having an active site as the radical polymerization starting point can also be introduced by reacting a hydroxyl group of the ring with a compound having a functional group such as amine, carboxylic acid, isocyanate, imidazole or acid anhydride to introduce another functional group except for the hydroxyl group and reacting it with the above organic halogen compound.

As the radically polymerizable compound used to introduce the side chain by radical polymerization, a compound having at least one group having an ethylenically unsaturated bond (to be referred to as "ethylenically unsaturated monomer" hereinafter), for example, a functional group such as (meth)acrylic group, vinyl group or styryl group is preferably used.

The following compounds are examples of the ethylenically unsaturated monomer.
Alkyl (meth)acrylates; methyl (meth)acrylate, ethyl (meth)acrylate, n-propyl (meth)acrylate, n-butyl (meth)acrylate, t-butyl (meth)acrylate and stearyl (meth)acrylate
Hydroxy (meth)acrylates; 2-hydroxyethyl (meth)acrylate and 2-hydroxypropyl (meth)acrylate
Cyano (meth)acrylates; cyanoethyl (meth)acrylate
Amino-based (meth)acrylates; (meth)acrylamide, N,N-dimethyl (meth)acrylamide, N-isopropyl (meth)acrylamide, N,N-dimethylaminoethyl (meth)acrylate and maleinimide (meth)acrylate
Fluoroalkyl (meth)acrylates; trifluoroethyl (meth)acrylate and pentafluorobutyl (meth)acrylate
Siloxanyl (meth)acrylates; tris(trimethylsiloxanyl)silylpropyl (meth)acrylate
Alkylene glycol polyol (meth)acrylates; ethylene glycol (meth)acrylate, triethylene glycol (meth)acrylate, polyethylene glycol (meth)acrylate, propylene glycol (meth)acrylate and polypropylene glycol (meth)acrylate
Aromatic vinyl compounds; styrene, p-methylstyrene, m-methoxystyrene and p-hydroxystyrene
Vinyl chlorides; sodium 4-vinylbenzoate and sodium p-styrene sulfonate
   Amphoteric ion (meth)acrylates; 2-methoxyacryloyloxyethyl phosphoryl choline and
   [2-(methacryloyloxy)ethyl]dimethyl(3-sulfopropyl)
ammonium hydroxide
Unsaturated monocarbons or esters thereof; cinnamic acid and crotonic acid
Oxirane compounds; glycidyl (meth)acrylate
Oxetane compounds; 2-oxetanemethyl (meth)acrylate
Unsaturated polycarboxylic acids (anhydrides); maleic acid (anhydride) and fumaric acid (anhydride)

Besides the ethylenically unsaturated monomers, oligomers or polymers having a terminal ethylenically unsaturated bond (to be also referred to as "macromonomers" hereinafter) may also be used.

Examples of the macromonomers are given below.
Polyethers; polyethylene oxide, polypropylene oxide and polytetramethylene oxide
Polyesters; polyethylene terephthalate and polycaprolactone
Polymers having a hydrocarbon main chain; polyethylene, polypropylene, polystyrene, polyvinyl methyl ether and poly(meth)acrylate
Polyamides; polyhexamethylene adipamide
Other polymers; polyimidic acid, polyimine amine, polyurethane, polyurea, polydimethyl siloxane and polycarbonate polymers
Copolymers of the polymers listed above

The above monomers or macromonomers may be used alone or in combination of two or more.

The above-described side chain having an appropriate size can be introduced by carrying out the radical polymerization, preferably living radical polymerization or atom-transfer radical polymerization of this radically polymerizable compound in the presence of the ring into which the above radical polymerization starting point has been introduced and adjusting the polymerization degree to a suitable range.

As understood from the above explanation, the side chain to be introduced into the ring of the cyclic compound may have a recurring unit produced by -O- bond, -NH- bond or -S- bond or substituent such as hydroxyl group, carboxyl group, acyl group, phenyl group, halogen atom, silyl group, mercapto group, vinyl group, NCO group or NCS group according to the introduction system.

Further, according to the type of the functional group of the compound used for the introduction of the side chain, part of the side chain may be bonded to a functional group of the ring of a cyclic molecule of another axial molecule to form a crosslinked structure.

It is preferred that a polymerizable functional group able to react with the polyurethane resin and/or precursor thereof (C) which will be described hereinafter should be introduced into the ring of the cyclic molecule in the polyrotaxane used in the present invention. Thereby, the compatibility with the polyurethane resin and/or precursor thereof (C) of the polyrotaxane is enhanced and further the photochromic compound is homogenously held in the photochromic layer obtained by polymerizing with the polyurethane resin and/or precursor thereof (C) while it is dispersed in the spaces of the polyrotaxane, whereby excellent photochromic properties can be developed continuously and the mechanical strength of the photochromic layer can be enhanced.

This polymerizable functional group is introduced by using the above side chain, and a suitable compound for forming the side chain is used to introduce the functional group.

This polymerizable functional group is preferably an OH group, SH group, NH₂ group, NCO group or NCS group. Out of these, the polymerizable functional group is most preferably an OH group. An electron absorbing group such as fluorine is introduced into the above side chain to control the electron density of the OH group, thereby making it possible to control the reaction rate of the OH group.

For example, the OH group, SH group or NH₂ group reacts with the NCO group or NCS group of the polyurethane resin and/or precursor thereof (C) to produce a urethane bond, thiourethane bond or urea bond whereas the NCO group or NCS group reacts with the OH group, SH group or NH₂ group of the polyurethane resin and/or precursor thereof (C).

The polyrotaxane (A) which is most preferably used in the present invention includes polyethylene glycol bonded to an adamantyl group at both ends as the axial molecule and cyclic molecules having a α-dextrin ring as the cyclic molecules, and the side chains (having a terminal OH group) are introduced into the ring by polycaprolactone.

### (B) Photochromic compound

As the photochromic compound having photochromic properties, photochromic compounds known per se may be used. They may be used alone or in combination of two or more.

Typical examples of the photochromic compounds include fulgide compounds, chromene compounds and spirooxazine compounds and are disclosed by many documents, for example, JP-A 2-28154, JP-A 62-288830, WO94/22850 and WO96/14596.

In the present invention, out of known photochromic compounds, from the viewpoints of photochromic properties such as color optical density, initial coloration, durability and fading speed, chromene compounds having an indeno(2,1-f)naphtho(1,2-b)pyran skeleton are preferably used, and chromene compounds having a molecular weight of not less than 540 are particularly preferably used as they are excellent especially in color optical density and fading speed.

The following chromene compounds are examples of the chromene compound which is particularly preferably used in the present invention.

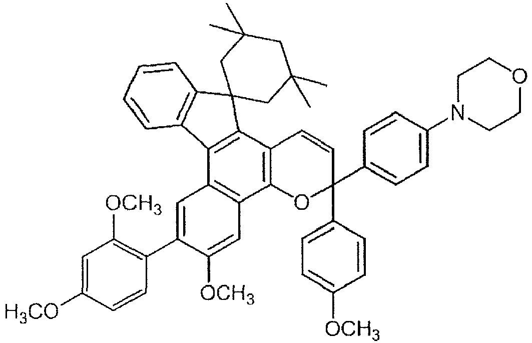

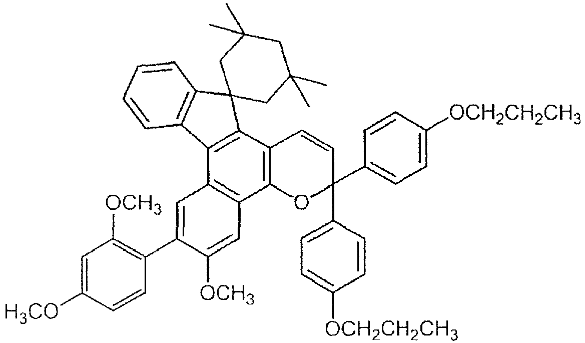

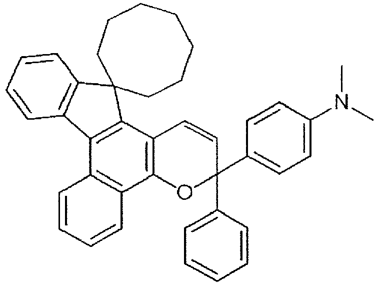

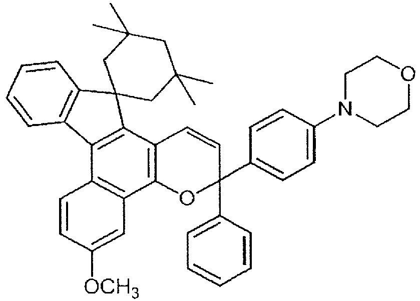

The photochromic coating composition of the present invention comprises (C2) a polyurethane resin precursor. A description is subsequently given of the polyurethane resin precursor (C2).

### (C2) polyurethane resin precursor

The polyurethane resin precursor (C2) in the present invention is a monomer component forming the above polyurethane resin and a prepolymer obtained by reacting the monomer component.

The monomer component used as the polyurethane resin precursor (C2) is selected from (C2-1) a polyiso (thio) cyanate compound having at least two isocyanate groups and/or isothiocyanate groups in one molecule, (C2-2) a poly(thi)ol compound having at least two hydroxyl groups and/or thiol groups in one molecule and (C2-3) a mono (thi) ol compound having one hydroxyl group or thiol group in one molecule. Specific examples thereof are given below.

A description is first given of (C2-1) the polyiso (thio) cyanate compound having at least two isocyanate groups and/or isothiocyanate groups in one molecule.

### <(C2-1) polyiso(thio)cyanate compound having at least two isocyanate groups and/or isothiocyanate groups in one molecule>

The polyiso(thio)cyanate compound having at least two isocyanate groups and/or isothiocyanate groups in one molecule (to be also simply referred to as "polyiso(thio)cyanate compound" hereinafter) which constitutes the photochromic coating composition of the present invention is a compound having at least two isocyanate groups and/or isothiocyanate groups in one molecule of the polyiso(thio)cyanate compound. Examples of the polyisocyanate compound out of the polyiso (thio) cyanate compounds include aliphatic isocyanates, alicyclic isocyanates, aromatic isocyanates, sulfur-containing aliphatic isocyanates, aliphatic sulfide-based isocyanates, aromatic sulfide-based isocyanates, aliphatic sulfone-based isocyanates, aromatic sulfone-based isocyanates, sulfonic acid ester-based isocyanates, aromatic sulfonic acid amide-based isocyanates and sulfur-containing heterocyclic isocyanates, and the like.

Examples of the polyisothiocyanate compound include aliphatic isothiocyanates, alicyclic isothiocyanates, aromatic isothiocyanates, heterocyclic-containing isothiocyanates, sulfur-containing aliphatic isothiocyanates, sulfur-containing aromatic isothiocyanates, sulfur-containing heterocyclic isothiocyanates, and the like..

The following compounds are given as examples of the polyiso(thio)cyanate compounds.

### Polyisocyanates:

Aliphatic isocyanates; ethylene diisocyanate, trimethylene diisocyanate, tetramethylene diisocyanate, hexamethylene diisocyanate, octamethylene diisocyanate, nonamethylene diisocyanate, 2,2'-dimethylpentane diisocyanate, 2,2,4-trimethylhexamethylene diisocyanate, decamethylene diisocyanate, butene diisocyanate, 1,3-butadiene-1,4-diisocyanate, 2,4,4-trimethylhexamethylene diisocyanate, 1,6,11-trimethylundecamethylene diisocyanate, 1,3,6-trimethylhexamethylene diisocyanate, 1,8-diisocyanato-4-isocyanatomethyl octane, 2,5,7-trimethyl-1,8-diisocyanato-5-isocyanatomethyl octane, bis(isocyanatoethyl)carbonate, bis(isocyanatoethyl)ether, 1,4-butylene glycol dipropyl ether 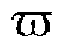 , 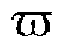 '-diisocyanate, lysine diisocyanatomethyl ester, lysine triisocyanate, 2-isocyanatoethyl-2,6-diisocyanato hexanoate and 2-isocyanatopropyl-2,6-diisocyanato hexanoate
Alicyclic isocyanates; isophorone diisocyanate, (bicyclo[2.2.1]heptane-2,5-diyl)bismethylene diisocyanate, (bicyclo[2.2.1]heptane-2,6-diyl)bismethylene diisocyanate, 2β,5α-bis(isocyanato)norbornane, 2β,5β-bis(isocyariato)norbornane, 2β,6α-bis(isocyanato)norbornane, 2β,6β-bis(isocyanato)norbornane, 2,6-di(isocyanatomethyl)furan, bis(isocyanatomethyl)cyclohexane, dicyclohexylmethane diisocyanate, 4,4-isopropylidenebis(cyclohexyl isocyanate), cyclohexane diisocyanate, methylcyclohexane diisocyanate, dicyclohexyl dimethylmethane diisocyanate, 2,2'-dimethyl dicyclohexylmethane diisocyanate, bis(4-isocyanato-n-butylidene)pentaerythritol, dimeric acid diisocyanate, 2-isocyanatomethyl-3-(3-isocyanatopropyl)-5-isocyanatomethyl-bicyclo[2.2.1]-heptane, 2-isocyanatomethyl-3-(3-isocyanatopropyl)-6-isocyanatomethyl-bicyclo[2.2.1]-heptane, 2-isocyanatomethyl-2-(3-isocyanatopropyl)-5-isocyanatomethyl-bicyclo[2.2.1]-heptane, 2-isocyanatomethyl-2-(3-isocyanatopropyl)-6-isocyanatomethyl-bicyclo[2.2.1]-heptane, 2-isocyanatomethyl-3-(3-isocyanatopropyl)-5-(2-isocyanatoethyl)-bicyclo[2.2.1]-heptane, 2-isocyanatomethyl-3-(3-isocyanatopropyl)-6-(2-isocyanatoethyl)-bicyclo[2.2.1]-heptane, 2-isocyanatomethyl-2-(3-isocyanatopropyl)-5-(2-isocyanatoethyl)-bicyclo[2.2.1]-heptane, 2-isocyanatomethyl-2-(3-isocyanatopropyl)-6-(2-isocyanatoethyl)-bicyclo[2.2.1]-heptane, 2,5-bis(isocyanatomethyl)-bicyclo[2.2.1]-heptane, 2,6-bis(isocyanatomethyl)-bicyclo[2.2.1]-heptane, 1,3,5-tris(isocyanatomethyl)cyclohexane, 3,8-bis(isocyanatomethyl)tricyclodecane, 3,9-bis(isocyanatomethyl)tricyclodecane, 4,8-bis(isocyanatomethyl)tricyclodecane, 4,9-bis(isocyanatomethyl)tricyclodecane, 1,5-diisocyanato decalin, 2,7-diisocyanto decalin, 1,4-diisocyanato decalin, 2,6-diisocyanato decalin, a mixture of bicyclo[4.3.0]nonane-3,7-diisocyanate and bicyclo[4.3.0]nonane-4,8-diisocyanate, a mixture of bicyclo[2.2.1]heptane-2,5-diisocyanate and bicyclo[2.2.1]heptane-2,6-diisocyanate, a mixture of bicyclo[2.2.2]octane-2,5-diisocyanate and bicyclo[2.2.2]octane-2,6-diisocyanate, and a mixture of tricyclo[5.2.1.0^{2.6}]decane-3,8-diisocyanate and tricyclo[5.2.1.0^{2.6}]decane-4,9-diisocyanate
Aromatic isocyanates; xylylene diisocyanate (o-, m-, p-), tetrachloro-m-xylylene diisocyanate, 4-chloro-m-xylylene diisocyanate, 4,5-dichloro-m-xylylene diisocyanate, 2,3,5,6-tetrabromo-p-xylylene diisocyanate, 4-methyl-m-xylylene diisocyanate, 4-ethyl-m-xylylene diisocyanate, bis(isocyanatoethyl)benzene, bis(isocyanatopropyl)benzene, 1,3-bis(α,α-dimethylisocyanatomethyl)benzene, 1,4-bis(α,α-dimethylisocyanatomethyl)benzene, α,α,α,α'-tetramethylxylylene diisocyanate, bis(isocyanatobutyl)benzene, bis(isocyanatomethyl)naphthalene, bis(isocyanatomethyl)diphenyl ether, bis(isocyanatoethyl)phthalate, mesitylene triisocyanate, 2,6-di(isocyanatomethyl)furan, phenylene diisocyanate, tolylene diisocyanate, ethyl phenylene diisocyanate, isopropyl phenylene diisocyanate, dimethyl phenylene diisocyanate, diethyl phenylene diisocyanate, diisopropyl phenylene diisocyanate, trimethylbenzene triisocyanate, benzene triisocyanate, 1,3,5-triisocyanatomethyl benzene, naphthalene diisocyanate, methyl naphthalene diisocyanate, biphenyl diisocyanate, tolidine diisocyanate, 4,4'-diphenylmethane diisocyanate, 3,3'-dimethyldiphenylmethane-4,4'-diisocyanate, bibenzyl-4,4'-diisocyanate, bis(isocyanatophenyl)ethylene, 3,3'-dimethoxybiphenyl-4,4'-diisocyanate, triphenylmethane triisocyanate, polymeric MDI, naphthalene triisocyanate, diphenylmethane-2,4,4'-triisocyanate, 3-methyldiphenylmethane-4,4' ,6-triisocyanate, 4-methyl-diphenylmethane-2,3,4' ,5,6-pentaisocyanate, phenyl isocyanatomethyl isocyanate, phenyl isocyanatoethyl isocyanate, tetrahydronaphthylene diisocyanate, hexahydrobenzene diisocyanate, hexahydrodiphenylmethane-4,4'-diisocyanate, diphenyl ether diisocyanate, ethylene glycol diphenyl ether diisocyanate, 1,3-propylene glycol diphenyl ether diisocyanate, benzophenone diisocyanate, diethylene glycol diphenyl ether diisocyanate, dibenzofuran diisocyanate, carbazole diisocyanate, ethyl carbazole diisocyanate and dichlorocarbazole diisocyanate
Sulfur-containing aliphatic isocyanates; thiodiethyl diisocyanate, thiodipropyl diisocyanate, thiodihexyl diisocyanate, dimethyl sulfone diisocyanate, dithiodimethyl diisocyanate, dithiodiethyl diisocyanate, 1-isocyanatomethylthio-2,3-bis (2-isocyanatoethylthio) propane, 1,2-bis(2-isocyanatoethylthio)ethane, 1,1,2,2-tetrakis(isocyanatomethylthio)ethane, 2,2,5,5-tetrakis (isocyanatomethylthio)-1,4-dithiane, 2,4-dithiapentane-1,3-diisocyanate, 2,4,6-trithiaheptane-3,5-diisocyanate, 2,4,7,9-tetrathiapentane-5,6-diisocyanate, bis(isocyanatomethylthio)phenyl methane, bis(isocyanatomethylthio)methane, bis(isocyanatoethylthio)methane, bis(isocyanatoethylthio)ethane, bis(isocyanatomethylthio)ethane and 1,5-isocyanato2-isocyanatomethyl-3-thiapentane
Aliphatic sulfide-based isocyanates; bis[2-(isocyanatomethylthio)ethyl]sulfide, dicyclohexyl sulfide-4,4'-diisocyanate, bis(isocyanatomethyl)sulfide, bis(isocyanatoethyl)sulfide, bis(isocyanatopropyl)sulfide, bis(isocyanatohexyl) sulfide, bis(isocyanatomethyl)disulfide, bis(isocyanatoethyl)disulfide and bis(isocyanatopropyl)disulfide
Aromatic sulfide-based isocyanates; diphenyl sulfide-2,4'-diisocyanate, diphenyl sulfide-4,4'-diisocyanate, 3,3'-dimethoxy-4,4'-diisocyanatodibenzyl thioether, bis(4-isocyanatomethylbenzene)sulfide, 4,4'-methoxybenzene thioethylene glycol-3,3'-diisocyanate, diphenyl disulfide-4,4'-diisocyanate, 2,2'-dimethyl diphenyl disulfide-5,5'-diisocyanate, 3,3'-dimethyl diphenyl disulfide-5,5'-diisocyanate, 3,3'-dimethyl diphenyl disulfide-6,6'-diisocyanate, 4,4'-dimethyl diphenyl disulfide-5,5'-diisocyanate, 3,3'-dimethoxy diphenyl disulfide-4,4'-diisocyanate and 4,4'-dimethoxydiphenyl disulfide-3,3'-diisocyanate
Aliphatic sulfone-based isocyanates; bis(isocyanatomethyl) sulfone
Aromatic sulfone-based isocyanates; diphenyl sulfone-4,4'-diisocyanate, diphenyl sulfone-3,3'-diisocyanate, benzylidene sulfone-4,4'-diisocyanate, diphenylmethane sulfone-4,4'-diisocyanate, 4-methyl diphenylmethane sulfone-2,4'-diisocyanate, 4,4'-dimethoxydiphenyl sulfone-3,3'-diisocyanate, 3,3'-dimethoxy-4,4'-diisocyanatodibenzyl sulfone, 4,4'-dimethyldiphenyl sufone-3,3'-diisocyanate, 4,4'-di-tert-butyldiphenyl sulfone-3,3'-diisocyanate, 4,4'-dimethoxybenzene ethylene disulfone-3,3'-diisocyanate and 4,4'-dichlorodiphenyl sulfone-3,3'-diisocyanate
Sulfonic acid ester-based isocyanates; 4-methyl-3-isocyanatobenzene sulfonyl-4'-isocyanatophenol ester and 4-methoxy-3-isocyanatobenzene sulfonyl-4'-isocyanatophenol ester
Aromatic sulfonic acid amide-based isocyanates; 4-methyl-3-isocyanatobenzene sulfonylanilide-3'-methyl-4'-isocyanate, dibenzene sulfonyl-ethylenediamine-4,4'-diisocyanate, 4,4'-dimethoxybenzene sulfonyl-ethylenediamine-3,3'-diisocyanate and 4-methyl-3-isocyanatobenzene sulfonylanilide-4-methyl-3'-isocyanate
Sulfur-containing heterocyclic isocyanates; thiophene-2,5-diisocyanate, thiophene-2,5-diisocyanato methyl, 1,4-dithiane-2,5-diisocyanate, 1,4-dithiane-2,5-diisocyanatomethyl, 1,3-dithiolane-4,5-diisocyanate, 1,3-dithiolane-4,5-diisocyanatomethyl, 1,3-dithiolane-2-methyl-4,5-diisocyanatomethyl, 1,3-dithiolane-2,2-diisocyanatoethyl, tetrahydrothiophene-2,5-diisocyanate, tetrahydrothiophene-2,5-diisocyanatomethyl, tetrahydrothiophene-2,5-diisocyantoethyl, tetrahydrothiophene-3,4-diisocyantomethyl, tricyclothiaoctane diisocyanate, 2-(1,1-diisocyanatomethyl)thiophene, 3-(1,1-diisocyanatomethyl)thiophene, 2-(2-thienylthio)-1,2-diisocyanatopropane, 2-(3-thienylthio)-1,2-diisocyanatopropane, 3-(2-thienyl)-1,5-diissocyanato-2,4-dithiapentane, 3-(3-thienyl)-1,5-diisocyanato-2,4-dithiapentane, 3-(2-thienylthio)-1,5-diisocyanato-2,4-dithiapentane, 3-(3-thienylthio)-1,5-diisocyanato-2,4-dithiapentane, 3-(2-thienylthiomethyl)-1,5-diisocyanato-2,4-dithiapentane, 3-(3-thienylthiomethyl)-1,5-diisocyanato-2,4-dithiapentane, 2,5-(diisocyanatomethyl)thiophene, 2,3-(diisocyanatomethyl)thiophene, 2,4-(diisocyanatomethyl)thiophene, 3,4-(diisocyanatomethyl)thiophene, 2,5-(diisocyanatomethylthio)thiophene, 2,3-(diisocyanatomethylthio)thiophene, 2,4-(diisocyanatomethylthio)thiophene, 3,4-(diisocyanatomethylthio)thiophene and 2,4-bisisocyanatomethyl-1,3,5-trithiane

Further, halogen substituents, alkyl substituents, alkoxy substituents, nitro substituents, polyhydric alcohol prepolymer type modified products, carbodiimide modified products, urea modified products and biuret modified products, and dimerization and trimerization reaction products of the above polyisocyanates may also be used.

### Polyisothiocyanates:

Aliphatic isothiocyanates; 1,2-diisothiocyanatoethane, 1,3-diisothiocyanatopropane, 1,4-diisothiocyanatobutane, 1,6-diisothiocyanatohexane and p-phenylene diisopropylidene diisothiocyanate
Alicyclic isothiocyanates; cyclohexyl isothiocyanate, cyclohexane diisothiocyanate, 2,4-bis(isothiocyanatomethyl)norbornane, 2,5-bis(isothiocyanatomethyl)norbornane, 3,4-bis(isothiocyanatomethyl)norbornane and 3,5-bis(isothiocyanatomethyl)norbornane
Aromatic isothiocyanates; phenyl isothiocyanate, 1,2-diisothiocyanatobenzene, 1,3-diisothiocyanatobenzene, 1,4-diisothiocyanatobenzene, 2,4-diisothiocyanatotoluene, 2,5-diisothiocyanato-m-xylene diisocyanate, 4,4'-diisothiocyanato-1,1'-biphenyl, 1,1'-methylenebis(4-isothiocyanatobenzene), 1,1'-methylenebis(4-isothiocyanato2-methylbenzene), 1,1'-methylenebis(4-isothiocyanato3-methylbenzene), 1,1'-(1,2-ethanediyl)bis(4-isothiocyanatobenzene), 4,4'-diisothiocyanatobenzophenone, 4,4'-diisothiocyanato-3,3'-dimethyl benzophenone, benzanilide-3,4'-diisothiocyanate, diphenyl ether-4,4'-diisothiocyanate and diphenylamine-4,4'-diisothiocyanate
Heterocyclic-containing isothiocyanates; 2,4,6-triisothiocyanato-1,3,5-triazine
Carbonyl isothiocyanates; hexanedioyl diisothiocyanate, nonanedioyl diisothiocyanate, carbonic diisothiocyanate, 1,3-benzene dicarbonyl diisothiocyanate, 1,4-benzene dicarbonyl diisothiocyanate and (2,2'-bipyridine)-4,4'-dicarbonyl diisothiocyanate

Further, polyfunctional isothiocyanates having at least one sulfur atom in addition to the sulfur atom of an isothiocyanate group may also be used. Examples of the polyfunctional isothiocyanates are given below.
Sulfur-containing aliphatic isothiocyanates; thiobis(3-isothiocyanatopropane), thiobis(2-isothiocyanatoethane) and dithiobis(2-isothiocyanatoethane)
Sulfur-containing aromatic isothiocyanates; 1-isothiocyanato4-{(2-isothiocyanato)sulfonyl}benzene, thiobis(4-isothiocyanatobenzene), sulfonyl bis(4-isothiocyanatobenzene), sulfinyl bis(4-isothiocyanatobenzene), dithiobis(4-isothiocyanatobenzene), 4-isothiocyanato-1-{(4-isothiocyanatophenyl)sulfonyl}-2-methoxy-benzene, 4-methyl-3-isothiocyanatobenzene sulfonyl-4'-isothiocyanatophenyl ester and 4-methyl-3-isothiocyanatobenzene sulfonylanilide-3'-methyl-4'-isothiocyanate
Sulfur-containing heterocyclic isothiocyanates; thiophene-2,5-diisothiocyanate and 1,4-dithiane-2,5-diisothiocyanate

### <preferred examples of component (C2-1)>

Preferred examples of the polyiso(thio)cyanate compound as the above component (C2-1) include pentamethylene diisocyanate, hexamethylene diisocyanate, heptamethylene diisocyanate, octamethylene diisocyanate, isophorone diisocyanate, norbornane diisocyanate, 2,5-bis(isocyanatomethyl)-bicyclo[2.2.1]-heptane, 2,6-bis(isocyanatomethyl)-bicyclo[2.2.1]-heptane, 1,2-bis(2-isocyanatoethylthio)ethane, xylene diisocyanate (o-, m-, p-), 2,4-tolylene diisocyanate, 2,6-tolylene diisocyanate, 4,4'-diphenylmethane diisocyanate and mixtures thereof.

A description is subsequently given of the poly (thi) ol compound having at least two hydroxyl groups and/or thiol groups in one molecule (C2-2).

### <(C2-2) poly(thi)ol compound having at least two hydroxyl groups and/or thiol groups in one molecule>

The poly (thi) ol compound having at least two hydroxyl groups and/or thiol groups in one molecule (to be also simply referred to as "poly(thi)ol compound" hereinafter) which constitutes the photochromic composition of the present invention is a compound having at least two hydroxyl groups (OH groups) and/or thiol groups (SH groups) in one molecule of the poly (thi) ol compound. Typical examples of the polyol compound out of the poly(thi)ol compounds include di-, tri-, tetra-, penta- and hexa-hydroxy compounds, polyesters having at least two OH groups in one molecule (or polyester polyols), polyethers having at least two OH groups in one molecule (or polyether polyols), polycarbonates having at least two OH groups in one molecule (or polycarbonate polyols), polycaprolactones having at least two OH groups in one molecules (or polycaprolactone polyols) and acrylic polymers having at least two OH groups in one molecule (or polyacrylic polyols).

Examples of the polythiol compound include aliphatic polythiols, aromatic polythiols, halogen-substituted aromatic polythiols, heterocyclic-containing polythiols, aromatic polythiols containing a sulfur atom in addition to a mercapto group, aliphatic polythiols containing a sulfur atom in addition to a mercapto group and heterocyclic-containing polythiols containing a sulfur atom in addition to a mercapto group.

Specific examples of these compounds are given below.
Aliphatic alcohols; ethylene glycol, diethylene glycol, propylene glycol, dipropylene glycol, butylene glycol, 1,5-dihydroxypentane, 1,6-dihydroxyhexane, 1,7-dihydroxyheptane, 1,8-dihydroxyoctane, 1,9-dihydroxynonane, 1,10-dihydroxydecane, 1,11-dihydroxyundecane, 1,12-dihydroxydodecane, neopentyl glycol, glycerin, trimethylolethane, trimethylolpropane, butane triol, 1,2-methyl glycoside, pentaerythritol, dipentaerythritol, tripentaerythritol, sorbitol, erythritol, threitol, ribitol, arabinitol, xylitol, allitol, mannitol, dorcitol, iditol, glycol, inositol, hexane triol, triglycerol, diglycerol, triethylene glycol, polyethylene glycol, tris(2-hydroxyethyl)isocyanurate, cyclobutanediol, cyclopentanediol, cyclohexanediol, cycloheptanediol, cyclooctanediol, cyclohexanedimethanol, hydroxypropyl cyclohexanol, tricyclo[5.2.1.0^{2.6}]decane-dimethanol, bicyclo[4,3,0]-nonanediol, cyclohexanediol, tricyclo[5,3,1,1^{3.9}] dodecanediol, bicyclo[4,3,0]nonanedimethanol, tricyclo [5,3,1,1^{3.9}]dodecane-diethanol, hydroxypropyl tricyclo[5,3,1,1^{3.9}]dodecanol, spiro[3,4]octanediol, butyl cyclohexanediol, 1,1'-bicyclohexylidene diol, cyclohexane triol, maltitol, lactitol, 3-methyl-1,5-dihydroxypentane, dihydroxyneopentyl, 2-ethyl-1,2-dihydroxyhexane, 2-methyl-1,3-dihydroxypropane, 1,4-cyclohexane dimethanol, 1,3-cyclohexane dimethanol, 1,2-cyclohexane dimethanol o-dihydroxy xylylene, m-dihydroxy xylylene, p-dihydroxy xylylene, 1,4-bis(2-hydroxyethyl)benzene, 1,4-bis(3-hydroxypropyl)benzene, 1,4-bis(4-hydroxybutyl)benzene, 1,4-bis(5-hydroxypentyl)benzene, 1,4-bis(6-hydroxyhexyl)benzene and 2,2-bis[4-(2"-hydroxyethyloxy)phenyl])propane
Aromatic alcohols; dihydroxy naphthalene, trihydroxy naphthalene, tetrahydroxy naphthalene, dihydroxy benzene, benzene triol, biphenyl tetraol, pyrogallol, (hydroxynaphthyl)pyrogallol, trihydroxy phenanthrene, bisphenol A, bisphenol F, xylylene glycol, tetrabromobisphenol A, bis(4-hydroxyphenyl)methane, 1,1-bis(4-hydroxyphenyl)ethane, 1,2-bis(4-hydroxyphenyl)ethane, bis(4-hydroxyphenyl)phenyl methane, bis(4-hydroxyphenyl)diphenyl methane, bis(4-hydroxyphenyl)-1-naphthyl methane, 1,1-bis(4-hydroxyphenyl)-1-phenyl ethane, 2-(4-hydroxyphenyl)-2-(3-hydroxyphenyl)propane, 2,2-bis(4-hydroxyphenyl)butane, 1,1-bis (4-hydroxyphenyl)butane, 2,2-bis(4-hydroxyphenyl)-3-methyl butane, 2,2-bis(4-hydroxyphenyl)pentane, 3,3-bis(4-hydroxyphenyl)pentane, 2,2-bis(4-hydroxyphenyl)hexane, 2,2-bis(4-hydroxyphenyl)octane, 2,2-bis(4-hydroxyphenyl)octane, 2,2-bis(4-hydroxyphenyl)-4-methyl pentane, 2,2-bis(4-hydroxyphenyl)heptane, 4,4-bis(4-hydroxyphenyl)heptane, 2,2-bis(4-hydroxyphenyl)tridecane, 2,2-bis(4-hydroxyphenyl)octane, 2,2-bis(3-methyl-4-hydroxyphenyl)propane, 2,2-bis(3-ethyl-4-hydroxyphenyl)propane, 2,2-bis(3-n-propyl-4-hydroxyphenyl)propane, 2,2-bis(3-isopropyl-4-hydroxyphenyl)propane, 2,2-bis(3-sec-butyl-4-hydroxyphenyl)propane, 2,2-bis(3-tert-butyl-4-hydroxyphenyl)propane, 2,2-bis(3-cyclohexyl-4-hydroxyphenyl)propane, 2,2-bis(3-allyl-4'-hydroxyphenyl)propane, 2,2-bis(3-methoxy-4-hydroxyphenyl)propane, 2,2-bis(3,5-dimethyl-4-hydroxyphenyl)propane, 2,2-bis(2,3,5,6-tetramethyl-4-hydroxyphenyl)propane, bis(4-hydroxyphenyl)cyanomethane, 1-cyano-3,3-bis(4-hydroxyphenyl)butane, 2,2-bis(4-hydroxyphenyl)hexafluoropropane, 1,1-bis(4-hydroxyphenyl)cyclopentane, 1,1-bis(4-hydroxyphenyl)cyclohexane, 1,1-bis(4-hydroxyphenyl)cycloheptane, 1,1-bis(3-methyl-4-hydorxyphenyl)cyclohexane, 1,1-bis(3,5-dimethyl-4-hydroxyphenyl)cyclohexane, 1,1-bis(3,5-dichloro-4-hydroxyphenyl)cyclohexane, 1,1-bis(3-methyl-4-hydroxyphenyl)-4-methyl cyclohexane, 1,1-bis(4-hydroxyphenyl)-3,3,5-trimethyl cyclohexane, 2,2-bis(4-hydroxyphenyl)norbornane, 2,2-bis(4-hydroxyphenyl)adamantane, 4,4'-dihydroxydiphenyl ether, 4,4'-dihydroxy-3,3'-dimethyldiphenyl ether, ethylene glycol bis(4-hydroxyphenyl)ether, 4,4'-dihydroxydiphenyl sulfide, 3,3'-dimethyl-4,4'-dihydroxydiphenyl sulfide, 3,3'-dicyclohexyl-4,4'-dihydroxydiphenyl sulfide, 3,3'-diphenyl-4,4'-dihydroxydiphenyl sulfide, 4,4'-dihydroxdiphenyl sulfoxide, 3,3'-dimethyl-4,4'-dihydroxydiphenyl sulfoxide, 4,4'-dihydroxidiphenyl sulfone, 4,4'-dihydroxy-3,3'-dimethyldiphenyl sulfone, bis(4-hydroxyphenyl)ketone, bis(4-hydroxy-3-methylphenyl)ketone, 7,7'-dihydroxy-3,3',4,4'-tetrahydro-4,4,4',4'-tetramethyl-2,2'-spirobi(2H-1-benzopyran), trans-2,3-bis(4-hydroxyphenyl)-2-butene, 9,9-bis(4-hydroxyphenyl)fluorene, 3,3-bis(4-hydroxyphenyl)-2-butanone, 1,6-bis(4-hydroxyphenyl)-1,6-hexanedione, 4,4'-dihydroxybiphenyl, hydroquinone and resorcin
Sulfur-containing polyols; bis-[4-(hydroxyethoxy)phenyl]sulfide, bis-[4-(2-hydroxypropoxy)phenyl]sulfide, bis-[4-(2,3-dihydroxypropoxy)phenyl]sulfide, bis-[4-(4-hydroxycyclohexyloxy)phenyl]sulfide, bis-[2-methyl-4-(hydroxyethoxy)-6-butylphenyl]sulfide, compounds obtained by adding an average of three or less molecules per hydroxyl group of ethylene oxide and/or propylene oxide to the above sulfur-containing polyols, di-(2-hydroxyethyl)sulfide, bis(2-hydroxyethyl)disulfide, 1,4-dithiane-2,5-diol, bis(2,3-dihydroxypropyl)sulfide, tetrakis(4-hydroxy-2-thiabutyl)methane, bis(4-hydroxyphenyl)sulfone, tetrabromobisphenol S, tetramethylbisphenol S, 4,4'-thiobis(6-tert-butyl-3-methylphenol) and 1,3-bis(2-hydroxyethylthioethyl)-cyclohexane
Sulfur-containing heterocyclic polyols; 2,5-bis(hydroxymethyl)-1,4-dithiane, 3-hydroxy-6-hydroxymethyl-1,5-dithiacycloheptane and 3,7-dihydroxy-1,5-dithiacyclooctane
Polyester polyols; compounds obtained from a condensation reaction between a polyol and a polybasic acid
Polyether polyols; compounds obtained from a reaction between a compound having at least two active hydrogen-containing groups in the molecule and an alkylene oxide, and modified products thereof
Polycaprolactone polyols; compounds obtained by the ring-opening polymerization of ε-caprolactone
Polycarbonate polyols; compounds obtained by the phosgenation of at least one low-molecular weight polyol, and compounds obtained by transesterification using ethylene carbonate, diethyl carbonate or diphenyl carbonate
Polyacrylic polyols; compounds obtained by the copolymerization of an acrylic acid ester or methacrylic acid ester containing a hydroxyl group and a monomer copolymerizable with these esters
Aliphatic polythiols; methanedithiol, 1,2-ethanedithiol, 1,1-propanedithiol, 1,2-propanedithiol, 1,3-propanedithiol, 2,2-propanedithiol, 1,6-hexanedithiol, 1,2,3-propanetrithiol, tetrakis(mercaptomethyl)methane, 1,1-cyclohexanedithiol, 1,2-cyclohexanedithiol, 2,2-dimethylpropane-1,3-dithiol, 3,4-dimethoxybutane-1,2-dithiol, 2-methylcyclohexane-2,3-dithiol, bicyclo[2,2,1]hepta-exo-cis-2,3-dithiol, 1,1-bis(mercaptomethyl)cyclohexane, thiomalic acid bis(2-mercaptoethyl ester), 2,3-dimercaptosuccinic acid (2-mercaptoethyl ester), 2,3-dimercapto-1-propanol(2-mercaptoacetate), 2,3-dimercapto-1-propanol(3-mercaptoacetate), diethylene glycol bis(2-mercaptoacetate), diethylene glycol bis(3-mercaptopropionate), 1,2-dimercaptopropylmethyl ether, 2,3-dimercaptopropylmethyl ether, 2,2-bis(mercaptomethyl)-1,3-propanedithiol, bis(2-mercaptoethyl)ether, ethylene glycol bis(2-mercaptoacetate), ethylene glycol bis(3-mercaptopropionate), 1,4-bis(3-mercaptobutyryloxy)butane, 1,4-butanediol bis(3-mercaptopropionate), 1,4-butanediol bis(thioglycolate), 1,6-hexanediol bis(thioglycolate), tetraethylene glycol bis(3-mercaptopropionate), trimethylolpropane tris(2-mercaptoacetate), trimethylolpropane tris(3-mercaptopropionate), trimethylolethane tris(3-mercaptobutyrate), trimethylolpropane tris(3-mercaptobutyrate), pentaerythritol tetrakis(2-mercaptoacetate), pentaerythritol tetrakis(3-mercaptopropionate), 1,2-bis(2-mercaptoethylthio)-3-mercaptopropane, dipentaerythritol hexakis(3-mercaptopropionate), pentaerythritol tetrakis(3-mercaptobutyrate), 1,4-bis(3-mercaptobutyryloxy)butane, trimethylolpropane tris(3-mercaptobutyrate), trimethylolethane tris(3-mercaptobutyrate), 1,2-bis[(2-mercaptoethyl)thio]-3-mercaptopropane, 2-mercaptomethyl-1,3-propanedithiol, 2-mercaptomethyl-1,4-butanedithiol, 2,4,5-tris(mercaptomethyl)-1,3-dithiolane, 2,2-bis(mercaptomethyl)-1,4-butanedithiol, 4,4-bis(mercaptomethyl)-3,5-dithiaheptane-1,7-dithiol, 2,3-bis(mercaptomethyl)-1,4-butanedithiol, 2,6-bis(mercaptomethyl)-3,5-dithiaheptane-1,7-dithiol, 4-mercaptomethyl-1,8-dimercapto-3,6-dithiaoctane, 2,5-bismercaptomethyl-1,4-dithiane, 1,1,3,3-tetrakis (mercaptomethylthio)propane, 5,7-dimercaptomethyl-1,11-dimercapto-3,6,9-trithiaundecane, 4,7-dimercaptomethyl-1, 11-dimercapto-3, 6, 9-trithiaundecane, 4,8-dimercaptomethyl-1,11-dimercapto-3, 6, 9-trithiaundecane and 4-mercaptomethyl-1,8-dimercapto-3,6-dithiaoctane.
Aromatic polythiols; 1,2-dimercaptobenzene, 1,3-dimercaptobenzene, 1,4-dimercaptobenzene, 1,2-bis(mercaptomethyl)benzene, 1,3-bis(mercaptomethyl)benzene, 1,4-bis(mercaptomethyl)benzene, 1,2-bis(mercaptoethyl)benzene, 1,3-bis(mercaptoethyl)benzene, 1,4-bis(mercaptoethyl)benzene, 1,2-bis(mercaptomethoxy)benzene, 1,3-bis(mercaptomethoxy)benzene, 1,4-bis(mercaptomethoxy)benzene, 1,2-bis(mercaptoethoxy)benzene, 1,3-bis(mercaptoethoxy)benzene, 1,4-bis(mercaptoethoxy)benzene, 1,2,3-trimercaptobenzene, 1,2,4-trimercaptobenzene, 1,3,5-trimercaptobenzene, 1,2,3-tris(mercaptomethyl)benzene, 1,2,4-tris(mercaptomethyl)benzene, 1,3,5-tris(mercaptomethyl)benzene, 1,2,3-tris(mercaptoethyl)benzene, 1,2,4-tris(mercaptoethyl)benzene, 1,3,5-tris(mercaptoethyl)benzene, 1,2,3-tris(mercaptomethoxy)benzene, 1,2,4-tris(mercaptomethoxy)benzene, 1,3,5-tris(mercaptomethoxy)benzene, 1,2,3-tris(mercaptoethoxy)benzene, 1,2,4-tris(mercaptoethoxy)benzene, 1,3,5-tris(mercaptoethoxy)benzene, 1,2,3,4-tetramercaptobenzene, 1,2,3,5-tetramercaptobenzene, 1,2,4,5-tetramercaptobenzene, 1,2,3,4-tetrakis(mercaptomethyl)benzene, 1,2,3,5-tetrakis (mercaptomethyl)benzene, 1,2,4,5-tetrakis(mercaptomethyl)benzene, 1,2,3,4-tetrakis(mercaptoethyl)benzene, 1,2,3,5-tetrakis(mercaptoethyl)benzene, 1,2,4,5-tetrakis(mercaptoethyl)benzene, 1,2,3,4-tetrakis(mercaptoethyl)benzene, 1,2,3,5-tetrakis(mercaptomethoxy)benzene, 1,2,4,5-tetrakis (mercaptomethoxy)benzene, 1,2,3,4-tetrakis(mercaptoethoxy)benzene, 1,2,3,5-tetrakis(mercaptoethoxy)benzene, 1,2,4,5-tetrakis(mercaptoethoxy)benzene, 2,2'-dimercaptobiphenyl, 4,4'-dimercaptobiphenyl, 4,4'-dimercaptobibenzyl, 2,5-toluenedithiol, 3,4-toluenedithiol, 1,4-naphthalenedithiol, 1,5-naphthalenedithiol, 2,6-naphthalenedithiol, 2,7-naphthalenedithiol, 2,4-dimethylbenzene-1,3-dithiol, 4,5-dimethylbenzene-1,3-dithiol, 9,10-anthracene dimethanethiol, 1,3-di(p-methoxyphenyl)propane-2,2-dithiol, 1,3-diphenylpropane-2,2-dithiol, phenylmethane-1,1-dithiol, 2,4-di(p-mercaptophenyl)pentane and 1,4-bis(mercaptopropylthiomethyl)benzene
Halogen-substituted aromatic polythiols; 2,5-dichlorobenzene-1,3-dithiol, 1,3-di(p-chlorophenyl)propane-2,2-dithiol, 3,4,5-tribromo-1,2-dimercaptobenzene and 2,3,4,6-tetrachloro-1,5-bis(mercaptomethyl)benzene
Heterocyclic-containing polythiols; 2-methylamino-4,6-dithiol sym-triazine, 2-ethylamino-4,6-dithiol sym-triazine, 2-amino-4,6-dithiol sym-triazine, 2-morpholino-4,6-dithiol sym-triazine, 2-cyclohexylamino-4,6-dithiol sym-triazine, 2-methoxy-4,6-dithiol sym-triazine, 2-phenoxy-4,6-dithiol sym-triazine, 2-thiobenzeneoxy-4,6-dithiol sym-triazine, 2-thiobutyloxy-4,6-dithiol sym-triazine and 1,3,5-tris(3-mercaptobutyryloxyethyl)-1,3,5-triazine-2,4,6(1H,3H,5H)-trione
Aromatic polythiols containing sulfur atom in addition to mercapto group; 1,2-bis(mercaptomethylthio)benzene, 1,3-bis(mercaptomethylthio)benzene, 1,4-bis(mercaptomethylthio)benzene, 1,2-bis(mercaptoethylthio)benzene, 1,3-bis(mercaptoethylthio)benzene, 1,4-bis(mercaptoethylthio)benzene, 1,2,3-tris(mercaptomethylthio)benzene, 1,2,4-tris(mercaptomethylthio)benzene, 1,3,5-tris(mercaptomethylthio)benzene, 1,2,3-tris(mercaptoethylthio)benzene, 1,2,4-tris(mercaptoethylthio)benzene, 1,3,5-tris(mercaptoethylthio)benzene, 1,2,3,4-tetrakis(mercaptomethylthio)benzene, 1,2,3,5-tetrakis (mercaptomethylthio)benzene, 1,2,4,5-tetrakis(mercaptomethylthio)benzene, 1,2,3,4-tetrakis(mercaptoethylthio)benzene, 1,2,3,5-tetrakis(mercaptoethylthio)benzene and 1,2,4,5-tetrakis(mercaptoethylthio)benzene
Aliphatic polythiols containing sulfur atom in addition to mercapto group; bis(mercaptomethyl)sulfide, bis(mercaptoethyl)sulfide, bis(mercaptopropyl)sulfide, bis(mercaptomethylthio)methane, bis(2-mercaptoethylthio)methane, bis(3-mercaptopropyl)methane, 1,2-bis(mercaptomethylthio)ethane, 1,2-(2-mercaptoethylthio)ethane, 1,2-(3-mercaptopropyl)ethane, 1,3-bis(mercaptomethylthio)propane, 1,3-bis(2-mercaptoethyl_thio)propane, 1,3-bis(3-mercaptopropylthio)propane, 1,2-bis(2-mercaptoethylthio)-3-mercaptopropane, 2-mercaptoethylthio-1,3-propanedithiol, 1,2,3-tris(mercaptomethylthio)propane, 1,2,3-tris(2-mercaptoethylthio)propane, 1,2,3-tris(3-mercaptopropylthio)propane, tetrakis(mercaptomethylthiomethyl)methane, tetrakis(2-mercaptoethylthiomethyl)methane, tetrakis(3-mercaptopropylthiomethyl)methane, bis(2,3-dimercaptopropyl)sulfide, 2,5-dimercapto-1,4-dithiane, bis(mercaptomethyl)disulfide, bis(mercaptoethyl)disulfide, bis(mercaptopropyl)disulfide, thioglycolic acid or mercaptopropionic acid esters of the above compounds, hydroxymethyl sulfide bis(2-mercaptoacetate), hydroxymethyl sulfide bis(3-mercaptopropionate), hydroxyethyl sulfide bis(2-mercaptoacetate), hydroxyethyl sulfide bis(3-mercaptopropionate), hydroxypropyl sulfide bis(2-mercaptoacetate), hydroxypropyl sulfide bis(3-mercaptopropionate), hydroxymethyl disulfide bis(2-mercaptoacetate), hydroxymethyl disulfide bis(3-mercaptopropionate), hydroxyethyl disulfide bis(2-mercaptoacetate), hydroxyethyl disulfide bis(3-mercaptopropionate), hydroxypropyl disulfide bis(2-mercaptoacetate), hydroxypropyl disulfide bis(3-mercaptopropionate), 2-mercaptoethyl ether bis(2-mercaptoacetate), 2-mercaptoethyl ether bis(3-mercaptopropionate), 1,4-dithiane-2,5-diol bis(2-mercaptoacetate), 1,4-dithiane-2,5-diol bis(3-mercaptopropionate), 2,5-bis(mercaptomethyl)-1,4-dithiane, 2,5-bis(2-mercaptoethyl)-1,4-dithiane, 2,5-bis(3-mercaptopropyl)-1,4-dithiane, 2-(2-mercaptoethyl)-5-mercaptomethyl-1,4-dithiane, 2-(2-mercaptoethyl)-5-(3-mercaptopropyl)-1,4-dithiane, 2-mercaptomethyl-5-(3-mercaptopropyl)-1,4-dithiane, thioglycolic acid bis(2-mercaptoethyl ester), thiodipropionic acid bis(2-mercaptoethyl ester), 4,4'-thiodibutyric acid bis(2-mercaptoethyl ester), dithiodiglycolic acid bis(2-mercaptoethy ester), dithiodipropionic acid bis(2-mercaptoethyl ester), 4,4'-dithiodibutyric acid bis(2-mercaptoethyl ester), thiodiglycolic acid bis(2,3-dimercaptopropyl ester), thiodipropionic acid bis(2,3-dimercaptopropyl ester), dithiodiglycolic acid bis(2,3-dimercaptopropyl ester), dithiodipropionic acid (2,3-dimercaptopropyl ester), 2-mercaptomethyl-6-mercapto-1,4-dithiacycloheptane, 4,5-bis(mercaptomethylthio)-1,3-dithiolane, 4,6-bis(mercaptomethylthio)-1,3-dithiane, 2-bis(mercaptomethylthio)methyl-1,3-dithietane, 2-(2,2-bis(mercaptomethylthio)ethyl)-1,3-dithietane, 1,2,7-trimercapto-4,6-dithiaheptane, 1,2,9-trimercapto-4,6,8-trithianonane, 1,2,11,-trimercapto-4,6,8,10-tetrathiaundecane, 1,2,13-trimercapto-4,6,8,10,12-pentathiatridecane, 1,2,8,9-tetramercapto-4,6-dithianonane, 1,2,10,11-tetramercapto-4,6,8-trithiaundecane, 1,2,12,13-tetramercapto-4,6,8,10-tetrathiatridecane, bis(2,5-dimercapto-4-thiapentyl)disulfide, bis(2,7-dimercapto-4,6-dithiaheptyl)disulfide, 1,2,5-trimercapto-4-thiapentane, 3,3-dimercaptomethyl-1,5-dimercapto-2,4-dithiapentane, 3-mercaptomethyl-1,5-dimercapto-2,4-dithiapentane, 3-mercaptomethylthio-1,7-dimercapto-2,6-dithiaheptane, 3,6-dimercaptomethyl-1,9-dimercapto-2,5,8-trithianonane, 3,7-dimercaptomethyl-1,9-dimercapto-2,5,8-trithianonane, 4,6-dimercaptomethyl-1,9-dimercapto-2,5,8-trithianonane, 3-mercaptomethyl-1,6-dimercapto-2,5-dithiahexane, 3-mercaptomethylthio-1,5-dimercapto-2-thiapentane, 1,1,2,2-tetrakis(mercaptomethylthio)ethane, 1,1,3,3-tetrakis(mercaptomethylthio)propane, 1,4,8,11-tetramercapto-2,6,10-trithiaundecane, 1,4,9,12-tetramercapto-2,6,7,11-tetrathiadodecane, 2,3-dithia-1,4-butanedithiol, 2,3,5,6-tetrathia-1,7-heptanedithiol, 2,3,5,6,8,9-hexathia-1,10-decanedithiol, 2-(1-mercapto-2-mercaptomethyl-3-thiabutyl)-1,3-dithiolane, 1,5-dimercapto-3-mercaptomethylthio-2,4-dithiapentane, 2-mercaptomethyl-4-mercapto-1,3-dithiolane, 2,5-dimercapto-1,4-dithiane, 2,6-dimercapto-1,4-dithiane, 2,4-dimercaptomethyl-1,3-dithietane, 1,2,6,10,11-pentamercapto-4,8-dithiaundecane, 1,2,9,10-tetramercapto-6-mercaptomethyl-4,7-dithiadecane, 1,2,9,13,14-pentamercapto-6-mercaptomethyl-4,7,11-trithiatetradecane, 1,2,6,10,14,15-hexamercapto-4,8,12-trithiapentadecane, 1,4-dithiane-2,5-bis(4,5-dimercapto-2-thiapentane) and 1,4-dithiane-2,5-bis(5,6-dimercapto-2,3-dithiahexane)
Heterocyclic-containing polythiols containing sulfur atom in addition to mercapto group; 3,4-thiophenedithiol, tetrahydrothiophene-2,5-dimercaptomethyl and 2,5-dimercapto-1,3,4-thiadiazole
Polythiols containing isocyanurate group; 1,2-bis[(2-mercaptoethyl)thio]-3-mercaptopropane, tris-{(3-mercaptopropionyloxy)-ethyl}-isocyanurate, 1,3,5-tris(3-mercaptobutyryloxyethyl)-1,3,5-triazine-2,4 ,6(1H,3H,5H)-trione and tris-[(3-mercaptopropionyloxy)-ethyl]-isocyanurate

As the above component (C2-2) in the present invention, a compound having at least one hydroxyl group and at least one thiol group in one molecule may also be used. Examples of the compound include the following compounds.
2-mercaptoethanol, 3-mercapto-1,2-propanediol, glycerin di(mercaptoacetate), 1-hydroxy-4-mercaptocyclohexane, 2,4-dimercaptophenol, 2-mercaptohydroquinone, 4-mercaptophenol, 1,3-dimercapto-2-propanol, 2,3-dimercapto-1-propanol, 1,2-dimercapto-1,3-butanediol, pentaerythritol tris(3-mercaptopropionate), pentaerythritol mono(3-mercaptopropionate), pentaerythritol bis(3-mercaptopropionate), pentaerythritol tris(thioglycolate), pentaerythritol pentakis(3-mercaptopropionate), hydroxymethyl-tris(mercaptoethylthiomethyl)methane, 1-hydroxyethylthio-3-mercaptoethylthiobenzene, 4-hydroxy-4'-mercaptodiphenyl sulfone, 2-(2-mercaptoethylthio)ethanol, dihydroxyethyl sulfide mono(3-mercaptopropionate), dimercaptoethane mono(salicylate) and hydroxyethylthiomethyl-tris(mercaptoethylthio)methane

A compound having a silsesquioxane structure may also be used as the component (C2-2). The silsesquioxane is a compound represented by the following formula (1).

(R¹-SiO_{3/2})ₙ (1)

{In the above formula, a plurality of R¹'s may be the same or different, each an organic group containing a hydroxyl group(s) and/or thiol group(s), hydrogen atom, alkyl group, cycloalkyl group, alkoxy group or phenyl group, and has an organic group containing at least two hydroxyl groups and/or thiol groups in one molecule, and the polymerization degree "n" is an integer of 6 to 100.}

The organic group containing a hydroxyl group(s) and/or thiol group(s) represented by R¹ in the above formula (1) is a monovalent hydrocarbon group having 1 to 10 carbon atoms to which at least one hydroxyl group and/or thiol group is bonded, or monovalent group containing an oxygen atom or sulfur atom in a chain having 1 to 10 carbon atoms to which at least one hydroxyl group and/or thiol group is bonded, and preferred examples thereof include alkylene chain having 1 to 10 carbon atoms and organic group derived from a polyol or polythiol.

The alkyl group represented by R¹ is preferably an alkyl group having 1 to 10 carbon atoms.

Examples of the alkyl group having 1 to 10 carbon atoms include methyl group, ethyl group, n-propyl group, isopropyl group, n-butyl group, sec-butyl group, tert-butyl group, n-pentyl group, n-hexyl group, n-octyl group and isooctyl group, and the like.

The cycloalkyl group is preferably a cycloalkyl group having 3 to 8 carbon atoms. Examples of the cycloalkyl group having 3 to 8 carbon atoms include cyclopropyl group, cyclobutyl group, cyclooctyl group, cyclohexyl group, cycloheptyl group, cyclooctyl group, and the like.

The alkoxy group is preferably an alkoxy group having 1 to 6 carbon atoms. Examples of the alkoxy group having 1 to 6 carbon atoms include methoxy group, ethoxy group, n-propoxy group, isopropoxy group, n-butoxy group, sec-butoxy group, tert-butoxy group, and the like.

In general, the silsesquioxane compound may take various structures such as cage-like, ladder-like and random structures. In the present invention, a mixture having a plurality of structures is preferred.

### <preferred examples of component (C2-2)>

The poly (thi) ol compound as the above component (C2-2) is preferably at least one compound selected from polyethylene polyol, polycaprolactone polyol, polycarbonate polyol, trimethylolpropane, pentaerythritol, trimethylolpropane tris(3-mercaptopropionate), pentaerythritol tetrakis(3-mercaptoproponate), dipentaerythritol hexakis(3-mercaptopropionate), tetraethylene glycol bis(3-mercaptopropionate), 1,4-butanediol bis(3-mercaptopropionate), 1,6-hexanediol bis(3-mercaptopropionate), 1,2-bis[(2-mercaptoethyl)thio]-3-mercaptopropane, 2,2-bis(mercaptomethyl)-1,4-butanedithiol, 1,4-bis(mercaptopropylthiomethyl)benzene, 2,5-bis(mercaptomethyl)-1,4-dithiane, 4-mercaptomethyl-1,8-dimercapto-3,6-dithiaoctane, 1,1,1,1-tetrakis(mercaptomethyl)methane, 1,1,3,3-tetrakis(mercaptomethylthio)propane, 1,1,2,2-tetrakis(mercaptomethylthio)ethane, 4,6-bis(mercaptomethylthio)-1,3-dithiane, 2-mercaptomethanol and tris-{(3-mercaptopropionyloxy)-ethyl}-isocyanurate.

A description is subsequently given of the mono (thi) ol compound having one hydroxyl group or thiol group in one molecule (C2-3).

### <(C2-3) mono(thi)ol compound having one hydroxyl group or thiol group in one molecule>

Preferably, the photochromic coating composition of the present invention comprises (C2-3) a mono(thi)ol compound having one hydroxyl group or thiol group in one molecule (to be also simply referred to as "mono(thi)ol compound" hereinafter) in addition to the above components (C2-1) and (C2-2). When the photochromic coating composition of the present invention is cured, a rigid cured body having a network structure with a (thio)urethane bond is obtained from a reaction between a polyiso(thio)cyanate compound and a poly(thi)ol compound. When the above component (C2-3) is further mixed with the photochromic composition, the mono(thi)ol compound having a free structure at one end is introduced into the network structure, thereby forming a flexible space around the mono(thi)ol compound. Therefore, it is assumed that the reversible structural change of the photochromic compound existent near this space is caused to occur swiftly, thereby making it possible to produce a photochromic laminate having excellent photochromic properties (color optical density, fading speed). By mixing the component (C2-3) with the photochromic coating composition of the present invention, high photochromic properties can be developed even when a small amount of the photochromic compound is used. Therefore, even when a photochromic compound having low solubility is used, a photochromic laminate which is fully practically usable can be obtained.

Since the mono(thi)ol compound has only one hydroxyl group or thiol group, the number of hydrogen bonds is smaller than that of the poly(thi)ol compound with the result that the viscosity of the photochromic coating composition can be reduced and coating performance to an optical substrate such as a plastic lens can be improved, thereby improving moldability.

Examples of the above mono (thi) ol compound used in the photochromic coating composition of the present invention include the following compounds.

Compounds having one hydroxyl group in one molecule; polyethylene glycol monooleyl ether, polyoxyethylene oleate, polyethylene glycol monolaurate, polyethylene glycol monostearate, polyethylene glycol mono-4-octylphenyl ether, linear polyoxyethylene alkyl ethers (polyethylene glycol monomethyl ether, polyoxyethylene lauryl ether, polyoxyethylene-2-ethylhexyl ether, polyoxyethylene tridecyl ether, polyoxyethylene cetyl ether, polyoxyethylene stearyl ether), and saturated alkyl alcohols having a linear or branched structure and 5 to 30 carbon atoms

Compounds having one thiol group in one molecule; 3-methoxybutyl thioglycolate, 2-ethylhexyl thioglycolate, 2-mercaptoethyloctanoic acid ester, 3-mercaptopropionic acid-3-methoxybutyl, 3-mercaptopropionic acid ethyl, 3-mercaptopropionic acid-2-octyl, n-octyl-3-mercaptopropionate, methyl-3-mercaptopropionate, tridecyl-3-mercaptopropionate, stearyl-3-mercaptopropionate, and saturated alkyl thiols having a linear or branched structure and 5 to 30 carbon atoms

Although the prepolymer is not particularly limited in the present invention, it is a product obtained by reacting the above components (C2-1) and the component (C2-2) in a certain ratio, preferably a compound having at least two OH groups, SH groups, NCO groups or NCS groups as polymerizable functional groups in the molecular chain. Thus, the compound having at least two polymerizable functional groups in the molecular chain is used as the prepolymer and a curing agent is added to prepare a two-liquid curable coating composition.

The above prepolymer is preferably a compound having a (thio)isocyanate group at both ends which is produced by using a poly(thio)isocyanate compound as the component (C2-1) and a poly(thi)ol compound as the component (C2-2), more preferably a compound obtained by further using a poly(thi)ol compound (C2-2) as the above curing agent. The components (C2-2) used in the prepolymer and used as a curing agent may be the same or different.

The above components (C2-1) to (C2-3) are used in combination to obtain physical properties of interest and at least two components (C2-1) and (C2-2) are preferably contained to form a photochromic layer.

As for the difference between (C1) and (C2), the polyurethane resin (C1) is synthesized and then mixed with the polyrotaxane (A) or the polyurethane resin precursor (C2) and the polyrotaxane (A) are mixed together. From the viewpoint of moldability, a composition comprising a mixture of the polyurethane resin precursor (C2) and the polyrotaxane (A) is preferably used.

The photochromic coating composition of the present invention may further comprise (D) a resin modifier, (E) a polymerization-curing accelerator, (F) an internal release agent and (G) an organic solvent to improve refractive index and moldability and adjust the hardness of the photochromic layer and the viscosity of the photochromic coating composition in addition to the above components (A), (B) and (C). A description is subsequently given of these components.

### (D) Resin modifier;

In the present invention, a resin modifier may be added to improve the refractive index of the obtained photochromic layer and adjust the hardness. Examples of the resin modifier include episulfide compounds, thietanyl compounds, polyamine compounds and epoxy compounds. Specific examples are described below.

### <episulfide compounds>

The episulfide compounds are compounds having at least two episulfide groups in one molecule and cured by ring-opening polymerization. The compounds may be added to obtain a high refractive index. Examples of the episulfide compounds are given below.

Bis(1,2-epithioethyl)sulfide, bis(1,2-epithioethyl)disulfide, bis(2,3-epithiopropyl)sulfide, bis(2,3-epithiopropylthio)methane, bis(2,3-epithiopropyl)disulfide, bis(2,3-epithiopropyldithio)methane, bis(2,3-epithiopropyldithio)ethane, bis(6,7-epithio-3,4-dithiaheptyl)sulfide, bis(6,7-epithio-3,4-dithiaheptyl)disulfide, 1,4-dithiane-2,5-bis(2,3-epithiopropyldithiomethyl), 1,3-bis(2,3-epithiopropyldithiomethyl)benzene, 1,6-bis(2,3-epithiopropyldithiomethyl)-2-(2,3-epithiopropyldithioethylthio)-4-thiahexane, 1,2,3-tris(2,3-epithiopropyldithio)propane, 1,1,1,1-tetrakis(2,3-epithiopropyldithiomethyl)methane, 1,3-bis(2,3-epithiopropyldithio)-2-thiapropane, 1,4-bis(2,3-epithiopropyldithio)-2,3-dithiabutane, 1,1,1-tris(2,3-epithiopropyldithio)methane, 1,1,1-tris(2,3-epithiopropyldithiomethylthio)methane, 1,1,2,2-tetrakis(2,3-epithiopropyldithio)ethane, 1,1,2,2-tetrakis(2,3-epithiopropyldithiomethylthio) ethane, 1,1,3,3-tetrakis(2,3-epithiopropyldithio)propane, 1,1,3,3-tetrakis(2,3-epithiopropyldithiomethylthio) propane, 2-[1,1-bis(2,3-epithiopropyldithio)methyl]-1,3-dithietane, and 2-[1,1-bis(2,3-epithiopropyldithiomethylthio)methyl]-1,3 -dithietane.

### <thietanyl compounds>

The thietanyl compounds are thietane compounds having at least two thietanyl groups in one molecule and cured by ring-opening polymerization. These compounds may be added to obtain a high refractive index. Some of the thietanyl compounds have an episulfide group together with a plurality of thietanyl groups and listed in the paragraph of the above episulfide compounds.

Other thietanyl compounds include metal-containing thietane compounds having a metal atom in the molecule and non-metal thietane compounds containing no metal. Examples of the thietanyl compounds are given below.

Non-metal thietane compounds; bis(3-thietanyl)disulfide, bis(3-thietanyl)sulfide, bis(3-thietanyl)trisulfide, bis(3-thietanyl)tetrasulfide, 1,4-bis(3-thietanyl)-1,3,4-trithiabutane, 1,5-bis(3-thietanyl)-1,2,4,5-tetrathiapentane, 1,6-bis(3-thietanyl)-1,3,4,6-tetrathiahexane, 1,6-bis(3-thietanyl)-1,3,5,6-tetrathiahexane, 1,7-bis(3-thietanyl)-1,2,4,5,7-pentathiaheptane, 1,7-bis(3-thietanylthio)-1,2,4,6,7-pentathiaheptane, 1,1-bis(3-thietanylthio)methane, 1,2-bis(3-thietanylthio)ethane, 1,2,3-tris(3-thietanylthio)propane, 1,8-bis(3-thietanylthio)-4-(3-thietanylthiomethyl)-3,6-dithiaoctane, 1,11-bis(3-thietanylthio)-4,8-bis(3-thietanylthiomethyl) -3,6,9-trithiaundecane, 1,11-bis(3-thietanylthio)-4,7-bis(3-thietanylthiomethyl) -3,6,9-trithiaundecane, 1,11-bis(3-thietanylthio)-5,7-bis(3-thietanylthiomethyl) -3,6,9-trithiaundecane, 2,5-bis(3-thietanylthiomethyl)-1,4-dithiane, 2,5-bis[[2-(3-thietanylthio)ethyl]thiomethyl]-1,4-dithiane, 2,5-bis(3-thietanylthiomethyl)-2,5-dimethyl-1,4-dithiane, bisthietanyl sulfide, bis(thietanylthio)methane, 3-[<(thietanylthio)methylthio>methylthio]thietane, bisthietanyl disulfide, bisthietanyl trisulfide, bisthietanyl tetrasulfide, bisthietanyl pentasulfide, 1,4-bis(3-thietanyldithio)-2,3-dithiabutane, 1,1,1-tris(3-thietanyldithio)methane, 1,1,1-tris(3-thietanyldithiomethylthio)methane, 1,1,2,2-tetrakis(3-thietanyldithio)ethane and 1,1,2,2-tetrakis(3-thietanyldithiomethylthio)ethane

### <metal-containing thietane compounds>

The thietane compounds contain the group 14 element such as Sn atom, Si atom, Ge atom or Pb atom; the group 4 element such as Zr atom or Ti atom; the group 13 element such as Al atom; or the group 12 element such as Zn atom as the metal atom in the molecule. The following compounds are particularly preferably used.
Alkylthio(thietanylthio)tin's; methylthio tris(thietanylthio)tin, ethylthio tris(thietanylthio)tin, propylthio tris(thietanylthio)tin and isopropylthio tris(thietanylthio)tin
Bis(alkylthio)bis(thietanylthio)tin's; bis(methylthio)bis(thietanylthio)tin, bis(ethylthio)bis(thietanylthio)tin, bis(propylthio)bis(thietanylthio)tin and bis(isopropylthio)bis(thietanylthio)tin
Alkylthio(alkylthio)bis(thietanylthio)tin's; ethylthio(methylthio)bis(thietanylthio)tin, methylthio(propylthio)bis(thietanylthio)tin, isopropylthio(methylthio)bis(thietanylthio)tin, ethylthio(propylthio)bis(thietanylthio)tin, ethylthio(isopropylthio)bis(thietanylthio)tin and isopropylthio(propylthio)bis(thietanylthio)tin
Bis(thietanylthio)cyclic dithiotin compounds; bis(thietanylthio)dithiastannetane, bis(thietanylthio)dithiastannolane, bis(thietanylthio)dithiastanninane and bis(thietanylthio)trithiastannocane
Alkyl(thietanylthio)tin compounds; methyl tris(thietanylthio)tin, dimethyl bis(thietanylthio)tin, butyl tris(thietanylthio)tin, tetrakis(thietanylthio)tin, tetrakis(thietanylthio)germanium and tris(thietanylthio)bismuth

### <polyamine compounds>

The polyamine compounds are compounds having at least two NH₂ groups in one molecule and form a urea bond through a reaction with a polyisocyanate or a thiourea bond through a reaction with a polyisothiocyanate. These monomers may be added to adjust the hardness. The following compounds are examples of the polyamine compound.

Ethylenediamine, hexamethylenediamine, isophoronediamine, nonamethylenediamine, undecamethylenediamine, dodecamethylenediamine, metaxylenediamine, 1,3-propanediamine, putrescine, 2-(2-aminoethylamino)ethanol, diethylenetriamine, p-phenylenediamine, m-phenylenediamine, melamine and 1,3,5-benzenetriamine

### <epoxy compounds>

The epoxy compounds have an epoxy group in the molecule as a polymerizable group and are cured by ring-opening polymerization. These compounds may be added to adjust the refractive index and the hardness of a lens. The epoxy-based compounds are roughly divided into aliphatic epoxy compounds, alicyclic epoxy compounds and aromatic epoxy compounds exemplified by the following compounds.

Aliphatic epoxy compounds; ethylene oxide, 2-ethyl oxirane, butyl glycidyl ether, phenyl glycidyl ether, 2,2'-methylene bisoxirane, 1,6-hexanediol diglycidyl ether, ethylene glycol diglycidyl ether, diethylene glycol diglycidyl ether, triethylene glycol diglycidyl ether, tetraethylene glycol diglycidyl ether, nonaethylene glycol diglycidyl ether, propylene glycol diglycidyl ether, dipropylene glycol diglycidyl ether, tripropylene glycol diglycidyl ether, tetrapropylene glycol diglycidyl ether, nonapropylene glycol diglycidyl ether, neopentyl glycol diglycidyl ether, trimethylolpropane triglycidyl ether, glycerol triglycidyl ether, diglycerol tetraglycidyl ether, pentaerythritol tetraglycidyl ether, diglycidyl ethers of tris(2-hydroxyethyl)isocyanurate and triglycidyl ethers of tris(2-hydroxyethyl)isocyanurate

Alicyclic epoxy compounds; isophoronediol diglycidyl ether and bis-2,2-hydroxycyclohexylpropane diglycidyl ether

Aromatic epoxy compounds; resorcin diglycidyl ether, bisphenol A diglycidyl ether, bisphenol F diglycidyl ether, bisphenol S diglycidyl ether, orthophthalic acid diglycidyl ester, phenol novolak polyglycidyl ether and cresol novolak polyglycidyl ether

Besides the above compounds, epoxy compounds having a sulfur atom in the molecule in addition to an epoxy group may also be used. The sulfur atom-containing epoxy compounds contribute especially to the improvement of refractive index and include chain aliphatic and cyclic aliphatic epoxy compounds exemplified by the following compounds.
Chain aliphatic sulfur atom-containing epoxy compounds; bis(2,3-epoxypropyl)sulfide, bis(2,3-epoxypropyl)disulfide, bis(2,3-epoxypropylthio)methane, 1,2-bis(2,3-epoxypropylthio)ethane, 1,2-bis(2,3-epoxypropylthio)propane, 1,3-bis(2,3-epoxypropylthio)propane, 1,3-bis(2,3-epoxypropylthio)-2-methylpropane, 1,4-bis(2,3-epoxypropylthio)butane, 1,4-bis(2,3-epoxypropylthio)-2-methylbutane, 1,3-bis(2,3-epoxypropylthio)butane, 1,5-bis(2,3-epoxypropylthio)pentane, 1,5-bis(2,3-epoxypropylthio)-2-methylpentane, 1,5-bis(2,3-epoxypropylthio)-3-thiapentane, 1,6-bis(2,3-epoxypropylthio)hexane, 1,6-bis(2,3-epoxypropylthio)-2-methylhexane, 3,8-bis(2,3-epoxypropylthio)-3,6-dithiaoctane, 1,2,3-tris(2,3-epoxypropylthio)propane, 2,2-bis(2,3-epoxypropylthio)-1,3-bis(2,3-epoxypropylthiomethyl)propane and 2,2-bis(2,3-epoxypropylthiomethyl)-1-(2,3-epoxypropylthio)butane
Cyclic aliphatic sulfur atom-containing epoxy compounds; 1,3-bis(2,3-epoxypropylthio)cyclohexane, 1,4-bis(2,3-epoxypropylthio)cyclohexane, 1,3 -bis(2,3-epoxypropylthiomethyl)cyclohexane, 1,4 -bis(2,3-epoxypropylthiomethyl)cyclohexane, 2,5 -bis(2,3-epoxypropylthiomethyl)-1,4-dithiane, 2,5 -bis(<2-(2,3-epoxypropylthio)ethyl>thiomethyl)-1,4-dithiane and
   2,5-bis(2,3-epoxypropylthiomethyl)-2,5-dimethyl-1,4-dithiane

The above resin modifiers (D) may be used alone or in combination of two or more, and the amount thereof is 1 to 30 parts by mass based on 100 parts by mass of the total of the components (A) and (C).

### (E) Polymerization-curing accelerator;

Various polymerization-curing accelerators may be used in the photochromic coating composition of the present invention to accelerate polymerization-curing according to the types of the above-described compounds.

For example, when the polymerization-curing accelerator is used for a reaction between hydroxyl group or thiol group and NCO group or NCS group, a urethane or urea reaction catalyst or a condensation agent is used as the polymerization-curing accelerator.

When an episulfide compound, thietanyl compound or epoxy compound is used, an epoxy curing agent or a cationic polymerization catalyst for the ring-opening polymerization of an epoxy group is used as the polymerization-curing accelerator.

### <urethane or urea reaction catalyst>

This reaction catalyst is used for the formation of a poly(thio)urethane bond through a reaction between a polyiso(thia)cyanate and a polyol or polythiol. Examples of this polymerization catalyst include tertiary amines, inorganic or organic salts corresponding to these, phosphine compounds, quaternary ammonium salts, quaternary phosphonium salts, Lewis acids and organic sulfonic acids.

Specific examples thereof are given below. When catalytic activity is too high according to the type of the selected compound, it can be controlled by using a mixture of a tertiary amine and a Lewis acid.
Tertiary amines; triethylamine, tri-n-propylamine, triisopropylamine, tri-n-butylamine, triisobutylamine, triethylamine, hexamethylene tetramine, N,N-dimethyl octylamine, N,N,N',N'-tetramethyl-1,6-diaminohexane, 4,4'-trimethylenebis(1-methylpiperidine) and 1,8-diazabicyclo-(5,4,0)-7-undecene
Phosphine compounds; trimethylphosphine, triethylphosphine, tri-n-propylphosphine, triisopropylphosphine, tri-n-butylphosphine, triphenylphosphine, tribenzylphosphine, 1,2-bis(diphenylphosphino)ethane and 1,2-bis(dimethylphosphino)ethane
Quaternary ammonium salts; tetramethylammonium bromide, tetrabutylammonium chloride and tetrabutylammonium bromide
Quaternary phosphonium salts; tetramethylphosphonium bromide, tetrabutylphosphonium chloride and tetrabutylphosphonium bromide
Lewis acids; triphenyl aluminum, dimethyltin dichloride, dimethyltin bis(isooctylthioglycolate), dibutyltin dichloride, dibutyltin dilaurate, dibutyltin maleate, dibutyltin maleate polymer, dibutyltin dilicinolate, dibutyltin bis(dodecylmercaptide), dibutyltin bis(isooctylthioglycolate), dioctyltin dichloride, dioctyltin maleate, dioctyltin maleate polymer, dioctyltin bis(butyl maleate), dioctyltin dilaurate, dioctyltin dilicinolate, dioctyltin dioleate, dioctyltin di(6-hydroxy)caproate, dioctyltin bis(isooctyl thioglycolate), didodecyltin dilicinolate, metal salts such as copper oleate, copper acetylacetonate, iron acetylacetonate, iron naphthenate, iron lactate, iron citrate, iron gluconate, potassium octanoate and 2-ethylhexyl titanate
Organic sulfonic acids; methane sulfonic acid, benzene sulfonic acid and p-toluene sulfonic acid

### <condensation agent>

Examples of the condensation agent are given below.
Inorganic acids; hydrogen chloride, hydrogen bromide, sulfuric acid and phosphoric acid
Organic acids; p-toluenesulfonic acid and camphorsulfonic acid
Acidic ion exchange resins; Amberlite and Amberlyst
Carbodiimides; dicyclohexyl carbodiimide and 1-ethyl-3-(3-dimethylaminopyrrolyl)-carbodiimide

### <epoxy curing agent>

Examples of the epoxy curing agent are given below.
Amine compounds and salts thereof; 2-methylimidazole, 2-ethyl-4-methylimidazole, 1,8-diazabicyclo(5,4,0)undecene-7-trimethylamine, benzyl dimethylamine, triethylamine, 2,4,6-tris(dimethylaminomethyl)phenol and 2-(dimethylaminomethyl)phenol
Quaternary ammonium salts; tetramethylammonium chloride, benzyltrimethylammonium bromide and tetrabutylammonium bromide
Organic phosphine compounds; tetra-n-butylphosphonium benzotriazolate and tetra-n-butylphosphonium-o,o-diethylphosphorodithioate
Metal carboxylic acid salts; chromium (III) tricarboxylate and tin octylate
Acetylacetone chelate compounds; chromium acetylacetonate

### <cationic polymerization catalyst>

Examples of the cationic polymerization catalyst are given below.
Lewis acid-based catalysts; BF₃·amine complex, PF₅, BF₃, AsF₅, SbF₅, and the like
Thermosetting cationic polymerization catalysts; phosphonium salts, quaternary ammonium salts, sulfonium salts, benzylammonium salts, benzylpyridinium salts, benzylsulfonium salts, hydrazinium salts, carboxylic acid esters, sulfonic acid esters and amine imides
Ultraviolet curable cationic polymerization catalysts; diaryl iodonium hexafluorophosphate and hexafluoroantimonic acid bis(dodecylphenyl)iodonium
The above polymerization-curing accelerators (E) may be used alone or in combination of two or more. The amount thereof may be so-called "catalytic amount", for example, 0.001 to 10 parts by mass, specifically 0.01 to 5 parts by mass based on 100 parts by mass of the total of the above components (A) and (C). When the photochromic coating composition comprises the component (D), the amount of the component (E) may be 0.001 to 10 parts by mass, specifically 0.01 to 5 parts by mass based on 100 parts by mass of the total of the components (A), (C) and (D).

### (F) internal release agent;

Any internal release agent may be used in the present invention if it has a mold releasing effect and does not impair the physical properties such as transparency of a resin, and surfactants are preferably used. Out of these, phosphate surfactants are particularly preferred. The internal release agent as used herein also includes those which have a mold releasing effect, for example, quaternary ammonium salts and quaternary phosphonium salts, out of the above catalysts. A suitable internal release agent is selected from these internal release agents from the viewpoints of combination with a monomer, polymerization conditions, economic efficiency and handling ease. Examples of the phosphate internal release agents are given below:
Mono-n-butyl phosphate, mono-2-ethylhexyl phosphate, mono-n-octyl phosphate, mono-n-butyl phosphate, bis(2-ethylhexyl)phosphate, di(2-ethylhexyl)phosphate, di-n-octyl phosphate, di-n-butyl phosphate

The above internal release agents (F) may be used alone or in combination of two or more, and the amount thereof may be small, for example, 0.001 to 10 parts by mass based on 100 parts by mass of the total of the components (A) and (C) . When the photochromic composition comprises the component (D), the amount is 0.001 to 10 parts by mass based on 100 parts by mass of the total of the components (A), (C) and (D) .

### (G) Organic solvent;

An organic solvent may be used in the photochromic coating composition of the present invention to improve the solubilities of the polyrotaxane (A), the photochromic compound (B) and the polyurethane resin and/or precursor thereof (C) and further the resin modifier (D), the polymerization-curing accelerator (E) and the internal release agent (F) which are preferably used and to reduce the viscosity of the obtained photochromic coating composition.

Examples of the organic solvent (G) used in the present invention include alcohols such as methanol, ethanol, n-propanol, i-propanol, n-butanol, t-butanol, 2-butanol and diacetone alcohol; polyhydric alcohol derivatives such as ethylene glycol monomethyl ether, ethylene glycol monoisopropyl ether, ethylene glycol monoethyl ether, ethylene glycol mono-n-propyl ether, ethylene glycol mono-n-butyl ether, ethylene glycol mono-t-butyl ether, propylene glycol monomethyl ether, polypropylene glycol monoethyl ether, propylene glycol-n-butyl ether and ethylene glycol dimethyl ether; ketones such as acetone, dimethyl ketone, methyl ethyl ketone, diethyl ketone, methyl isobutyl ketone, di-n-propyl ketone, di-i-propyl ketone and acetyl acetone; aromatic hydrocarbons such as benzene, toluene and xylene; aliphatic hydrocarbons such as hexane and heptane, acetates such as methyl acetate, ethyl acetate, propyl acetate, butyl acetate, 2-methyl ethyl acetate and 2-ethoxyethyl acetate; dioxane; dimethyl formamide (DMF); dimethyl sulfoxide (DMSO); tetrahydrofuran (THF); cyclohexanone; and halogenated hydrocarbons such as chloroform and dichloromethane. A suitable organic solvent is selected from these according to the types of the components (A) to (C) and further the components (D) to (F), the material of the plastic lens substrate and coating ease.

The above organic solvents (G) may be used alone or in combination of two or more, and the amount thereof may be 10 to 1,000 parts by mass based on 100 parts by mass of the total of the components (A) and (C). When the photochromic coating composition comprises the component (D), the amount of the organic solvent is 10 to 1,000 parts by mass based on 100 parts by mass of the total of the components (A), (C) and (D).

### Other compounding components;

As long as the effect of the present invention is not impaired, the photochromic coating composition of the present invention may be optionally mixed with compounding agents known per se, for example, stabilizers and additives such as release agent, ultraviolet absorbent, infrared absorbent, ultraviolet stabilizer, antioxidant, discoloration inhibitor, antistatic agent, fluorescent dye, dye, pigment and aroma chemical and further a thiol such as t-dodecylmercaptan as a polymerization control agent.

When an ultraviolet stabilizer is used, the durability of the photochromic compound can be further improved advantageously. As the ultraviolet stabilizer, there are known hindered amine optical stabilizers, hindered phenol antioxidants and sulfur-containing antioxidants. Particularly preferred ultraviolet stabilizers are given below.

bis(1,2,2,6,6-pentamethyl-4-piperidyl)sebacate; the ADK STAB LA-52, LA-57, LA-62, LA-63, LA-67, LA-77, LA-82, LA-87 of Adeka Corporation;
2,6-di-t-butyl-4-methyl-phenol; 2,6-ethylenebis(oxyethylene)bis[3-(5-t-butyl-4-hydroxy-m -tolyl)propionate]; and the IRGANOX 1010, 1035, 1075, 1098, 1135, 1141, 1222, 1330, 1425, 1520, 259, 3114, 3790, 5057 and 565 of Ciba Specialty Chemicals.

Although the amount of the ultraviolet stabilizer is not particularly limited as long as the effect of the present invention is not impaired, generally, it is 0.1 to 10 parts by mass, specifically 0.5 to 5 parts by mass based on 100 parts by mass of the total of the polyrotaxane (A) and the polyurethane resin and/or precursor thereof (C).

### <preferred composition of photochromic coating composition>

In the photochromic coating composition of the present invention which comprises the above polyrotaxane (A), the photochromic compound (B) and the polyurethane resin and/or precursor thereof (C) as essential components, in general, the photochromic compound (B) is used in an amount of 0.1 to 10 parts by mass, particularly preferably 0.5 to 5 parts by mass based on 100 parts by mass of the total of the polyrotaxane (A) and the polyurethane resin and/or precursor thereof (C).

The amount of the polyrotaxane (A) is preferably 0.5 to 50 parts by mass, more preferably 1 to 20 parts by mass based on 100 parts by mass of the total of the polyrotaxane (A) and the polyurethane resin and/or precursor thereof (C) .

Further, when the resin modifier (D) is used, the amount of the polyrotaxane (A) is preferably 0.5 to 50 parts by mass, more preferably 1 to 20 parts by mass based on 100 parts by mass of the total of the polyrotaxane (A), the polyurethane resin and/or precursor thereof (C) and the resin modifier (D).

In the present invention, to develop the photochromic property and moldability improving effect of the polyrotaxane (A) to the maximum, it is most preferred that the polymerizable functional group to be introduced into the side chains of the polyrotaxane (A) should be an OH group and/or SH group and the components (C2-1) to (C2-3) which are the polyurethane resin precursors (C2) should be suitably combined to form a urethane bond, thiourethane bond, urea bond or thiourea bond, specifically urethane bond or thiourethane bond. As for the preferred ratio of these components, from the viewpoints of photochromic properties and moldability, the ratio of (A)/(C2-1)/(C2-2)/(C2-3) is 0.5 to 50/20 to 74/20 to 74/0 to 40 parts by mass, more preferably 1 to 20/25 to 69/23 to 67/0 to 20 parts by mass based on 100 parts by mass of the total of the components (A), (C2-1), (C2-2) and (C2-3).

Since a photochromic laminate having excellent photochromic properties (color optical density, fading speed) can be produced by mixing the component (C2-3), the ratio of (A) / (C2-1) / (C2-2) / (C2-3) is preferably 0.5 to 50/20 to 74/20 to 74/2 to 40 parts by mass, more preferably 1 to 20/25 to 69/23 to 67/2 to 20 parts by mass.

In this case, the amounts of the SH group and the OH group are each 0.8 to 1.2 moles, preferably 0.85 to 1.15 moles, most preferably 0.9 to 1.1 moles based on 1 mol of the NCO group or NCS group.

### <use of photochromic coating composition>

The photochromic coating composition of the present invention which comprises at least the above polyrotaxane (A), the photochromic compound (B) and the polyurethane resin and/or precursor thereof (C) is used.

A photochromic layer is formed by adding the organic solvent (G) to the above photochromic coating composition as required to adjust the viscosity of a coating solution, applying this coating solution to a plastic lens, and drying and/or curing it, thereby making it possible to develop photochromic properties. Stated more specifically, when the polyurethane resin (C1) is used as the component (C), preferably, the organic solvent (G) is used to prepare a photochromic coating composition, the photochromic coating composition is then applied to a plastic lens, and the organic solvent is dried off. When the polyurethane resin precursor (C2) is used as (C) and the organic solvent (G) is used in combination, the obtained photochromic coating composition is dried and cured and when the organic solvent (G) is not used, only curing should be performed.

Polymerization-curing for the manufacture of the photochromic laminate of the present invention is performed by carrying out a polycondensation reaction and a polyaddition reaction with heat.

The optical substrate used in the present invention is not particularly limited if it is a substrate having optical transparency, and glass and plastic lenses and known lens substrates such as window glass for houses and automobiles may be used but plastic lenses are particularly preferably used.

As the plastic lens of the present invention, known lenses which are now used as plastic lenses, for example, thermoplastic resin lenses such as (meth)acrylic resin and polycarbonate-based resin lenses; and crosslinkable resin lenses such as polyfunctional (meth)acrylic resin, allyl resin, thiourethane resin, urethane resin and thioepoxy resin lenses may be used.

Preferably, the photochromic coating composition of the present invention provides a photochromic layer on the optical substrate, for example, a plastic lens by the lamination method. Stated more specifically, the photochromic coating composition of the present invention is dissolved in an organic solvent as required to prepare a coating solution which is then applied to the surface of the optical substrate such as a plastic lens substrate by spin coating. When the organic solvent is used, it is removed by drying and then polymerization-curing is carried out by heating to form a photochromic layer on the surface of the optical substrate (coating method).

When the coating method is employed, the viscosity (23°C) of the photochromic coating composition is not particularly limited but preferably 20 to 5,000 mPa.s (cP) more preferably 70 to 1,000 mPa.s (cP). When the viscosity is too high, the coating temperature should be raised.

When the photochromic layer is to be formed on the surface of the optical substrate by the above lamination method (coating method, adhesion between the photochromic layer and the optical substrate can be enhanced by subjecting the surface of the optical substrate to a chemical treatment with an alkaline solution or acidic solution, or physical treatment by corona discharge, plasma discharge or polishing. As a matter of course, a transparent adhesive resin layer may be formed on the surface of the optical substrate.

In the present invention, the thickness of the photochromic layer in the obtained laminate is not particularly limited but preferably 10 to 100 µm, more preferably 20 to 70 µm.

To produce the laminate, the photochromic layer should be formed on the optical substrate by the following method that is employed to cure a coating film of the photochromic coating composition. A coating film of the photochromic coating composition is first formed on the optical substrate by the coating method or cast polymerization method. Then, the optical substrate having the coating film is left at a temperature of 20 to 150°C to cure or polymerize and cure the coating film.

The above-described photochromic coating composition of the present invention can develop excellent photochromic properties such as color optical density and fading speed and is effectively used for the manufacture of an optical substrate provided with photochromic properties, for example, a photochromic lens with high moldability without producing an appearance defect such as a crack or strain.

The laminate obtained by the above method has a poly(thio)urethane coating film which is able to have high moldability and excellent photochromic properties. Therefore, it can have excellent surface hardness.

The photochromic layer formed from the photochromic coating composition of the present invention may be subjected to post-processing such as dyeing with a dispersion dye, the formation of a hard coat layer by using a hard coat agent comprising a silane coupling agent or a silicon, zirconium, antimony, aluminum, tin or tungsten sol as the main component, or antireflection treatment or antistatic treatment by forming a thin film by depositing a metal oxide such as SiO₂, TiO₂ or ZrO₂ or a thin coating film of an organic polymer according to purpose.

### EXAMPLES

The following examples are provided for the purpose of further illustrating the present invention but are in no way to be taken as limiting. In the following examples and comparative examples, the above components and photochromic properties were evaluated by the following methods.

### (A) Polyrotaxane;

RX-1: polyrotaxane having side chains with a hydroxyl group and an average molecular weight of about 600 and a weight average molecular weight of 700,000

### <RX-1 preparation method>

The method of preparing RX-1 as the component (A) is described below.

### (1-1)Preparation of PEG-COOH;

Linear polyethylene glycol (PEG) having a molecular weight of 35,000 was prepared as a polymer for forming an axial molecule.

### Formulation;

10 g of PEG, 100 mg of TEMPO (2,2,6,6-tetramethyl-1-piperidinyloxy radical) and 1 g of sodium bromide were dissolved in 100 ml of water.

5 ml of a commercially available sodium hypochlorate aqueous solution (effective chlorine content of 5 %) was added to this solution and stirred at room temperature for 10 minutes. Thereafter, a maximum of 5 ml of ethanol was added to terminate the reaction. After extraction was made by using 50 ml of methylene chloride, methylene chloride was distilled off, the extract was dissolved in 250 ml of ethanol, and re-precipitation was carried out at -4°C for 12 hours to collect and dry PEG-COOH.

### (1-2)Preparation of polyrotaxane;

3 g of PEG-COOH prepared above and 12 g of α-cyclodextrin (a-CD) were each dissolved in 50 ml of 70°C hot water, and the obtained solutions were fully mixed together by shaking. Then, this mixed solution was reprecipitated at 4°C for 12 hours, and the precipitated inclusion complex was freeze-dried and collected. After 0.13 g of adamantanamine was dissolved in 50 ml of dimethyl formamide (DMF) at room temperature, the above inclusion complex was added to and fully mixed with the resulting solution quickly by shaking. Subsequently, a solution prepared by dissolving 0.38 g of a BOP reagent (benzotriazol-1-yl-oxy-tris(dimethylamino)phosphonium hexafluorophosphate) in DMF was further added and fully mixed by shaking. Further, a solution prepared by dissolving 0.14 ml of diisopropylethylamine in DMF was added and fully mixed by shaking to obtain a slurry reagent. The slurry reagent obtained above was left to stand at 4°C for 12 hours. Thereafter, 50 ml of a DMF/methanol mixed solvent (volume ratio of 1/1) was added to and mixed with the reagent, the obtained mixture was then centrifuged, and the supernatant was thrown away. After the resulting product was washed with the above DMF/methanol mixed solution, it was washed by using methanol and centrifuged to obtain a precipitate. After the obtained precipitate was vacuum dried, it was dissolved in 50 ml of DMSO, and the obtained transparent solution was added dropwise to 700 ml of water to precipitate a polyrotaxane. The precipitated polyrotaxane was collected by centrifugation and vacuum dried. Further, the polyrotaxane was dissolved in DMSO, precipited in water, collected and dried to obtain a purified polyrotaxane. The inclusion amount of α-CD was 0.25.

The inclusion amount was calculated by dissolving the polyrotaxane in DMSO-d₆ and measuring with a ¹H-NMR measuring instrument (JNM-LA500 of JEOL Ltd.) in accordance with the following method.

X, Y and X/(Y-X) mean the following.
X: integrated value of proton derived from hydroxyl group of 4 to 6 ppm of cyclodextrin
Y: integrated value of proton derived from methylene chains of 3 to 4 ppm of cyclodextrin and PEG
X/(Y-X): proton ratio of cyclodextrin to PEG

X/(Y-X) when the maximum inclusion amount is 1 theoretically is first calculated and then compared with X/(Y-X) calculated from the analytical value of the actual compound to calculate the inclusion amount.

### (1-3)Introduction of side chains into polyrotaxane;

500 mg of the above purified polyrotaxane was dissolved in 50 ml of a 1 mol/L NaOH aqueous solution, and 3.83 g (66 mmol) of propylene oxide was added to the resulting solution and stirred in an argon atmosphere at room temperature for 12 hours. Then, the above polyrotaxane solution was neutralized to a pH of 7 to 8 by using a 1 mol/L HCl aqueous solution, dialyzed with a dialysis tube and freeze-dried to obtain a hydroxypropylated polyrotaxane.

The modification degree of the OH groups of the cyclic molecule by the hydroxypropyl group was 0.5. A mixed solution was prepared by dissolving 5 g of the obtained hydroxypropylated polyrotaxane in 30 g of ε-caprolactone at 80°C. After this mixed solution was stirred at 110°C for 1 hour while dry nitrogen was blown, 0.16 g of a 50 wt% xylene solution of tin(II) 2-ethylhexanoate was added to this mixed solution and stirred at 130°C for 6 hours. Thereafter, xylene was added to obtain a polycaprolactone-modified polyrotaxane xylene solution having a nonvolatile content of about 35 mass% into which side chains have been introduced.

The polycaprolactone-modified polyrotaxane xylene solution prepared above was added dropwise to hexane, collected and dried to obtain a side chain-modified polyrotaxane (A) having OH groups as polymerizable functional groups. When the obtained polyrotaxane was identified by ¹H-NMR and GPC, it was confirmed that it was a polyrotaxane having a desired structure.

The physical properties of this polyrotaxane (A) were as follows.
Side chain modification degree: 0.5
Molecular weight of side chain: about 600 on average Weight average molecular weight Mw of polyrotaxane (GPC): 700,000
RX-2: polyrotaxane having side chains with a hydroxyl group and an average molecular weight of about 500 and a weight average molecular weight of 400,000

The polyrotaxane RX-2 was obtained in the same manner as the above RX-1 except that PEG having a molecular weight of 20,000 was used.

The physical properties of this polyrotaxane (A) were as follows.
Inclusion amount of α-CD: 0.25
Side chain modification degree: 0.5
Molecular weight of side chain: about 500 on average Weight average molecular weight Mw of polyrotaxane (GPC): 400,000
RX-4: polyrotaxane having side chains with a thiol group and an average molecular weight of about 500 and a weight average molecular weight of 450,000

1.27 g (0.012 mol) of 3-mercaptopropionic acid, 50 g of toluene and 0.34 g (0.002 mol) of p-toluene sulfonic acid were added to 10 g of RX-2 obtained above in a nitrogen atmosphere to carry out a reaction under reflux for 6 hours. Water produced by the reaction was co-boiled with a solvent, only water was removed to the outside of the system with a separator, and the solvent returned to a reactor. After the reaction product was added dropwise to hexane in a nitrogen atmosphere and precipitated, a solid was collected and dried to obtain RX-4. The physical properties of this polyrotaxane (A) were as follows.
Inclusion amount of α-CD: 0.25
Side chain modification degree: 0.5
Molecular weight of side chain: about 600 on average Weight average molecular weight Mw of polyrotaxane (GPC): 450,000

GPC measurement conditions were as follows. A liquid chromatograph (manufactured by Nihon Waters K.K.) was used as an apparatus for GPC measurement. The Shodex GPC KF-805 (elimination limit molecule quantity: 2,000,000) of Showa Denko K.K. was used as a column. Dimethyl formamide (DMF) was used as a developing solution to measure at a flow rate of 1 ml/min and a temperature of 40°C. Polystyrene was used as a reference standard to obtain the weight average molecular weight by comparative conversion. A differential refractometer was used as a detector.

### Photochromic compound (B);

PC1:

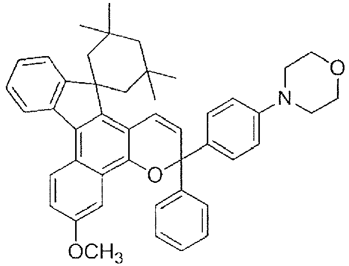

Polyurethane resin precursor (C2) ; (C2-1) polyiso(thio)cyanate compound having at least two isocyanate groups and/or isothiocyanate groups in one molecule
XDI: m-xylene diisocyanate
IPDI: isophorone diisocyanate
NBDI: (bicyclo[2.2.1]heptane-2,5(2,6)-diyl)bismethylene diisocyanate
(C2-2) poly(thi)ol compound having at least two hydroxyl groups and/or thiol groups in one molecule Polyol;
PL1: DURANOL of Asahi Kasei Chemicals Co., Ltd. (polycarbonate diol, number average molecular weight of 500)
PL2: DURANOL of Asahi Kasei Chemicals Co., Ltd. (polycarbonate diol, number average molecular weight of 800)
PE1: polyether diol, number average molecular weight of 400
TMP: trimethylolpropane

### Polythiol;

TMMP: trimethylolpropane tris(3-mercaptopropionate)
(C2-3) mono(thi)ol compound having one hydroxyl group or thiol group in one molecule
PELE23: polyoxyethylene lauryl ether (n≒23, Mw=1198)
PGMS25: polyethylene glycol monostearate (n≒25, Mw=1386)
MP-70: polypropylene glycol monomethyl ether manufactured by Kao Corporation (Mw=439)

Other compounding components
(D) resin modifier: CE1 isophorone diamine
(E) polymerization-curing accelerator;
DBTD: dibutyltin laurate

### (A) Organic solvent

DMF: dimethyl formamide
MEK: methyl ethyl ketone

### Polyurethane resin (C)

(C1) PU1: polyurethane resin

### Method of producing PU1 (C1)

30 g of IPDI (C2-1), 27 g of PEI (C2-2) and 240 mL of DMF (G) were fed to a three-necked flask having a stirring blade, cooling tube, thermometer and nitrogen gas introduction pipe to carry out a reaction in a nitrogen atmosphere at 120°C for 5 hours and then cooled to 25°C, 10.2 g of IPDA as the component (D) was added dropwise to the resulting reaction product to carry out a reaction at 25°C for 1 hour, and the solvent was distilled off under reduced pressure to obtain PU1 (C-1). When the molecular weight of the obtained PU1 was measured by GPC, it was 10,000 in terms of polyoxyethylene. Absorption derived from an isocyanate group at the end of the molecule was confirmed at around 2250 cm⁻¹ by infrared absorption spectral measurement.

### Example 1

A homogeneous solution (photochromic coating composition) was prepared by mixing together the following components in accordance with the following formulation. The amount of each component is shown in Table 1. The initial viscosity of the obtained coating composition was measured with the RST rheometer (RSTCPS) of BROOKFIELD and shown in Table 1.

### Formulation;

(A) Polyrotaxane: 0.5 part by mass of RX-1
(B) Photochromic compound: 0.4 part by mass of PC1
(C2-1) polyiso(thio)cyanate compound having at least two isocyanate groups and/or isothiocyanate groups in one molecule: 4.9 parts by mass of IPDI
(C2-2) poly(thi)ol compound having at least two hydroxyl groups and/or thiol groups in one molecule: 2.1 parts by mass of PL1, 1.7 parts by mass of TMP
(C2-3) mono(thi)ol compound having one hydroxyl group or thiol group in one molecule: 0.8 part by mass of PELE23

### (E) curing accelerator: 0.0001 part by mass of DBTD

A photochromic laminate was obtained by using the above photochromic coating composition in accordance with the following method.

A thiourethane-based plastic lens having a center thickness of about 2 mm, a spherical power of -6.00D and a refractive index of 1.60 was prepared as an optical substrate. This thiourethane-based plastic lens was subjected to 5 minutes of alkali etching at 50°C by using a 10 % sodium hydroxide aqueous solution and then fully rinsed with distilled water in advance.

The photochromic coating composition was dropped on the surface of the above plastic lens which was turned at 2, 000 rpm with a spin coater (1H-DX2 of MIKASA) . Thereafter, the plastic lens was heated at 120°C for 1 hour to polymerize and cure the photochromic coating composition so as to obtain a photochromic laminate. The thickness of the photochromic layer was about 30 µm.

The obtained photochromic laminate exhibited photochromic properties such as a maximum absorption wavelength of 595 nm, a color optical density of 0.85 and a fading speed of 50 seconds. As for moldability, the laminate had an optical strain of 1, no appearance defect, a Vickers hardness of 13 and an adhesion of 100. The maximum absorption wavelength, color optical density, fading speed, moldability, Vickers hardness and adhesion were evaluated by the following methods.

### [evaluation items]

(1) Maximum absorption wavelength (λmax) : Maximum absorption wavelength after color development obtained with the spectrophotometer (instantaneous multi-channel photodetector MCPD1000) of Otsuka Electronics Co., Ltd. by using the obtained photochromic laminate as a sample and exposing it to light having a beam intensity at 365 nm of 2.4 mW/cm² on the surface of the laminate and at 245 nm of 24 µW/cm² with the L-2480 (300W) SHL-100 xenon lamp of Hamamatsu Photonics K.K. through an aero-mass filter (of Corning Incorporated) at 20°C±1°C for 120 seconds. The maximum absorption wavelength is connected with color at the time of color development.
(2) Color optical density {ε (120) - ε(0)}: Difference between absorbance {ε(120)} after 120 seconds of exposure to light at the above maximum absorption wavelength and absorbance ε(0) before exposure. It can be said that as this value becomes larger, photochromic properties become more excellent. Color which was developed outdoors was evaluated visually.
(3) Fading speed [tl/2 (sec.)]: Time elapsed until the absorbance at the above maximum absorption wavelength of a sample drops to 1/2 of {ε(120) - ε(0)} when exposure is continued for 120 seconds and then stopped. It can be said that as this time becomes shorter, photochromic properties become more excellent.
(4) Moldability: The optical strain and appearance defect such as a crack of the manufactured photochromic laminate were evaluated by the following methods.
   Optical strain; The spherical power (SPH1, unit of diopter) of a plastic lens before the formation of a photochromic layer and the spherical power (SPH2, unit of diopter) of the plastic lens after the formation of the photochromic layer were measured by using the LM-1800PD auto lens meter (manufactured by Nidec) to evaluate the optical strain of the lens by the difference ΔSPH=|SPH2-SPH1|. The evaluation criteria are given below.
   1: ΔSPH of less than 0.10
   2: ΔSPH of not less than 0.10
   Appearance defect; 10 photochromic laminates were manufactured to count the number of photochromic laminates having an appearance defect such as a crack.
(5) Vickers hardness: The Vickers hardness of the obtained photochromic layer was measured with the PMT-X7A micro-Vickers hardness meter (of Matsuzawa Co., Ltd.). A square pyramid type diamond indenter was used to carry out the evaluation of Vickers hardness under a load of 10 gf for an indenter retention time of 30 seconds. After this measurement was made 4 times in total, an average value of three measurement data excluding a first one with a large measurement error was given.
(6) Adhesion
   Adhesion was evaluated by a cross-cut tape test in accordance with JISD-0202. That is, a cutter knife was used to make cuts in the surface of the photochromic layer of the obtained photochromic laminate at intervals of about 1 mm so as to form 100 squares. A cellophane adhesive tape (Cellotape (registered trademark) of Nichiban Co., Ltd.) was strongly attached to the surface and then peeled off at a stretch in a 90° direction from the surface to count the number of squares left behind of the photochromic layer.

### Examples 2 to 8 and 12, Comparative Examples 1 and 2

Photochromic laminates were manufactured and evaluated in the same manner as in Example 1 except that photochromic coating compositions shown in Tables 1 and 2 were used. The results are shown in Table 3.

### Example 9

(A) Polyrotaxane: 0.5 part by mass of RX-2
(B) Photochromic compound: 0.4 part by mass of PC1
(C2-1) polyiso(thio)cyanate compound having at least two isocyanate groups and/or isothiocyanate groups in one molecule: 4.9 parts by mass of IPDI
(C2-2) poly(thi)ol compound having at least two hydroxyl groups and/or thiol groups in one molecule: 2.1 parts by mass of PL1, 1.7 parts by mass of TMP
(C2-3) mono(thi)ol compound having one hydroxyl group or thiol group in one molecule: 0.8 part by mass of PELE23
(E) curing accelerator: 0.0001 part by mass of DBTD

An allyl resin plastic lens CR39 having a center thickness of about 2 mm, a spherical power of -2.00D and a refractive index of 1.50 was prepared as an optical substrate. This CR39 was subjected to 5 minutes of alkali etching at 50°C by using a 10 % sodium hydroxide aqueous solution and fully rinsed with distilled water in advance.

The photochromic coating composition was dropped on the surface of the above plastic lens which was turned at 2,000 rpm with a spin coater (1H-DX2 of MIKASA) . Thereafter, the plastic lens was heated at 120°C for 1 hour to polymerize and cure the photochromic coating composition so as to obtain a photochromic laminate. The thickness of the photochromic layer was about 30 µm.

The obtained photochromic laminate exhibited photochromic properties such as a maximum absorption wavelength of 595 nm, a color optical density of 0.86 and a fading speed of 49 seconds. As for moldability, the photochromic laminate had an optical strain of 1, no appearance defect, a Vickers hardness of 13 and an adhesion of 100. The composition of the coating composition is shown in Table 2 and the physical properties of the obtained photochromic laminate are shown in Table 3.

### Reference Example 10

(A) Polyrotaxane: 0.5 part by mass of RX-2
(B) Photochromic compound: 0.4 part by mass of PC1
(C1-1) polyurethane resin: 3 parts by mass of PU1
(C2-2) poly(thi)ol compound having at least two hydroxyl groups and/or thiol groups in one molecule: 1.5 parts by mass of TMP
(E) curing accelerator: 0.0001 part by mass of DBTD
(G) organic solvent: 10 parts by mass of MEK

A photochromic laminate was obtained by using the above photochromic coating composition in accordance with the following method.

A thiourethane-based plastic lens having a center thickness of about 2 mm, a spherical power of -6.00D and a refractive index of 1. 60 was prepared as an optical substrate. This thiourethane-based plastic lens was subjected to 5 minutes of alkali etching at 50°C by using a 10 % sodium hydroxide aqueous solution and fully rinsed with distilled water in advance.

The photochromic coating composition was dropped on the surface of the above plastic lens which was turned at 1,000 rpm with a spin coater (1H-DX2 of MIKASA). Thereafter, the plastic lens was heated at 120°C for 1 hour to polymerize and cure the photochromic coating composition so as to obtain a photochromic laminate. The thickness of the photochromic layer was about 40 µm.

The obtained photochromic laminate exhibited photochromic properties such as a maximum absorption wavelength of 595 nm, a color optical density of 0.89 and a fading speed of 40 seconds. As for moldability, the photochromic laminate had an optical strain of 1, no appearance defect, a Vickers hardness of 10 and an adhesion of 100. The composition of the coating composition is shown in Table 2 and the physical properties of the obtained photochromic laminate are shown in Table 3.

### Reference Example 11

A photochromic laminate was manufactured and evaluated in the same manner as in Example 10 except that a photochromic coating composition shown in Table 2 was used. The results are shown in Table 3.

**Table 1**

| No. | Component (A) (pbm) | Component (B) (pbm) | Component (C2) (pbm) | | | Component (E) (pbm) | viscosity (cP) | optical substrate |
|---|---|---|---|---|---|---|---|---|
| | | | (C2-1) (pbm) | (C2-2) (pbm) | (C2-3) (pbm) | | | |
| Ex.1 | RX-1 (0.5) | PC1 (0.4) | IPDI (4.9) | TMP/PL1 (1.7/2.1) | PELE23 (0.8) | DBTD (0.0001) | 800 | Thiourethane-based plastic lens |
| Ex.2 | RX-1 (1.4) | PC1 (0.4) | IPDI (5.1) | TMP/PL1 (1.9/1.6) | - | DBTD (0.0001) | 1500 | Thiourethane-based plastic lens |
| Ex.3 | RX-1 (0.07) | PC1 (0.4) | IPDI (4.9) | TMP/PL1 (1.7/2.53) | PELE23 (0.8) | DBTD (0.0001) | 300 | Thiourethane-based plastic lens |
| Ex.4 | RX-1 (2.5) | PC1 (0.4) | IPDI (5.1) | TMP/PL1 (1.9/0.5) | - | DBTD (0.0001) | 2500 | Thiourethane-based plastic lens |
| Ex.5 | RX-2 (0.6) | PC1 (0.4) | XDI (4.4) | TMP/PL1 (1.8/2.3) | PELE23 (0.9) | DBTD (0.0001) | 400 | Thiourethane-based plastic lens |
| Ex. 6 | RX-2 (0.5) | PC1 (0.4) | NBDI (4.9) | TMP/PL2 (1.7/2.1) | PGMS25 (0.8) | DBTD (0.0001) | 300 | Thiourethane-based plastic lens |
| Ex.7 | RX-2 (0.5) | PC1 (0.4) | NBDI (4.9) | TMMP/PL2 (1.7/2.1) | PGMS25 (0.8) | DBTD (0.0001) | 250 | Thiourethane-based plastic lens |
| C.Ex.1 | - | PC1 (0.4) | IPDI (5.8) | TMP/PL1 (2.0/2.2) | - | DBTD (0.0001) | 150 | Thiourethane-based plastic lens |
| C.Ex.2 | - | PC1 (0.4) | IPDI (4.7) | TMP/PL1 (1.5/1.9) | PELE23 (1.9) | DBTD (0.0001) | 100 | Thiourethane-based plastic lens |

| | | | | | | | | |
|---|---|---|---|---|---|---|---|---|
| Ex.: Example C.Ex.: Comparative Example pbm: parts by mas | | | | | | | | |

**Table 2**

| No. | Compo. (A) (pbm) | Compo. (B) (pbm) | Compo. (C1) (pbm) | Component (C2) (pbm) | | | Compo. (D) (pbm) | Compo. (E) (pbm) | Compo. (G) (pbm) | Viscosity (cP) | optical substrate |
|---|---|---|---|---|---|---|---|---|---|---|---|
| | | | | (C2-1) (pbm) | (C2-2) (pbm) | (C2-3) (pbm) | | | | | |
| Ex. 8 | RX-4 (0.5) | PC1 (0.4) | - | XDI (4.9) | TMMP/PL1 (1.7/2.1) | PGMS25 (0.8) | - | DBTD (0.0001) | - | 200 | Thiourethane-based plastic lens |
| Ex. 9 | RX-2 (0.5) | PC1 (0.4) | - | IPDI (4.9) | TMP/PL1 (1.7/2.1) | PELE23 (0.8) | - | DBTD (0.0001) | - | 350 | Aryl resin plastic lens |
| Ex. 10 | RX-2 (0.5) | PC1 (0.4) | PU1 (3) | IPDI (4) | TMP (1.5) | MP-70 (1) | - | DBTD (0.0001) | MEK (10) | 1000 | Thiourethane-based plastic lens |
| Ex. 11 | RX-2 (0.5) | PC1 (0.4) | PU1 (5) | I PDI (3) | - | - | CE1 (1.5) | - | MEK (10) | 2000 | Thiourethane-based plastic lens |
| Ex. 12 | RX-2 (1.3) | PC1 (0.4) | - | NBDI (4.8) | TMP/PL1 (1.7/1.4) | PGMS25 (0.8) | - | DBTD (0.0001) | | 1000 | Thiourethane-based plastic lens |

| | | | | | | | | | | | |
|---|---|---|---|---|---|---|---|---|---|---|---|
| Ex.: Example pbm: parts by mass Compo.:Component | | | | | | | | | | | |

**Table 3**

| No. | Photochromic properties | | | moldability | | Vickers hardness | adhesion |
|---|---|---|---|---|---|---|---|
| | Maximum absorption wavelength (λmax) | color optical density | fading speed(sec) | optical strain | appearance defect | | |
| Ex.1 | 595 | 0.85 | 50 | 1 | 0 | 13 | 100 |
| Ex.2 | 595 | 0.87 | 48 | 1 | 0 | 12 | 100 |
| Ex.3 | 595 | 0.79 | 65 | 1 | 0 | 15 | 100 |
| Ex.4 | 595 | 0.87 | 42 | 1 | 0 | 7 | 100 |
| Ex. 5 | 597 | 0.83 | 53 | 1 | 0 | 15 | 100 |
| Ex.6 | 595 | 0.85 | 50 | 1 | 0 | 13 | 100 |
| Ex.7 | 596 | 0.85 | 50 | 1 | 0 | 13 | 100 |
| C.Ex.1 | 595 | 0.12 | 340 | 2 | 4 | 16 | 100 |
| C.Ex.2 | 595 | 0.86 | 53 | 1 | 0 | 3 | 100 |
| C.Ex.3 | 595 | 0.82 | 70 | 2 | 2 | 7 | 0 |
| C.Ex.4 | 595 | 0.82 | 72 | 2 | 2 | 7 | 100 |
| Ex.8 | 598 | 0.82 | 51 | 1 | 0 | 14 | 100 |
| Ex.9 | 595 | 0.86 | 49 | 1 | 0 | 12 | 100 |
| Ex.10 | 595 | 0.89 | 40 | 1 | 0 | 6 | 100 |
| Ex.11 | 595 | 0.75 | 67 | 1 | 0 | 15 | 100 |
| Ex.12 | 595 | 0.90 | 40 | 1 | 0 | 9 | 100 |

| | | | | | | | |
|---|---|---|---|---|---|---|---|
| Ex.: Example C.Ex.: Comparative Example | | | | | | | |

### Comparative Example 3

A polyrotaxane (RX-3) having an acrylic group as a reactive group was prepared by the following method.

After 0.01 g of dibutyl hydroxy toluene (polymerization inhibitor) was added to 30 g of a polycaprolactone-modified polyrotaxane xylene solution prepared in the same manner as the above polyrotaxane RX-1 preparation method (1-3), 3.8 g of 2-acryloyloxyethyl isocyanate was added dropwise to the resulting solution. This solution was stirred at 40°C for 16 hours to obtain a polyrotaxane xylene solution having an acrylic group introduced into the end of polycaprolactone. This polyrotaxane xylene solution was added dropwise to hexane, collected and dried to prepare a polyrotaxane having an acrylic group as a reactive group (RX-3). The inclusion amount of α-CD was 0.25, the modification degree was 0.5, the molecular weight of each of the side chains of the obtained polyrotaxane was about 600, and the weight average molecular weight Mw of the polyrotaxane was 950,000.

Then, 0.5 part by mass of RX-3, 2 parts by mass of polyethylene glycol diacrylate (average chain length of ethylene glycol chains of 9, average molecular weight of 508), 3 parts by mass of trimethylolpropane trimethacrylate, 4 parts by mass of 2,2-bis[4-(methacryloyloxypolyethoxy)phenyl]propane (average chain length of ethylene glycol chains of 10, average molecular weight of 804), 0.1 part by mass of glycidyl methacrylate, 0.4 part by mass of PC1 and 0.04 part by mass of phenylbis(2,4,6-trimethylbenzoyl)-phosphine oxide (trade name: Irgacure 819, manufactured by BASF) as a polymerization initiator were added and fully mixed together under agitation to obtain a photochromic coating composition.

A photochromic layer was formed by the following method.

A thiourethane-based plastic lens having a center thickness of about 2 mm, a spherical power of -6.00D and a refractive index of 1.60 was prepared as an optical substrate. This thiourethane-based plastic lens was subjected to 5 minutes of alkali etching at 50°C by using a 10 % sodium hydroxide aqueous solution and fully rinsed with distilled water in advance.

The photochromic coating composition obtained above was dropped on the surface of the above plastic lens which was turned at 1,000 rpm with a spin coater (1H-DX2 of MIKASA) . Thereafter, the lens coated with the photochromic coating composition was exposed to light by using a metal halide lamp having an out of 200 mW/cm² in a nitrogen gas atmosphere for 90 seconds to cure the coating film. Then, the lens was further heated at 110°C for 1 hour to obtain a laminate having a photochromic layer. The thickness of the photochromic layer was about 30 µm.

The photochromic properties such as maximum absorption wavelength, color optical density and fading speed, moldability, Vickers hardness and adhesion of the obtained photochromic laminate were evaluated in the same manner as in Example 1.

The results are shown in Table 3.

### Comparative Example 4

A photochromic laminate was manufactured and evaluated in the same manner as in Comparative Example 3 except that a moisture-curable primer (trade name; TR-SC-P, manufactured by Tokuyama Corporation) layer having a thickness of about 10 µm was formed between the plastic lens and the photochromic layer. The results are shown in table 3.

As obvious from the above Examples and Comparative Examples, the photochromic laminates obtained by forming a layer of the photochromic coating composition of the present invention are excellent in photochromic properties, moldability, surface hardness and adhesion. The photochromic laminates of Examples 1 to 7 were excellent in photochromic properties, moldability, surface hardness and adhesion whereas the photochromic laminate of Comparative Example 1 was unsatisfactory in terms of photochromic properties and moldability and the photochromic laminate of Comparative Example 2 was unsatisfactory in terms of surface hardness though it had satisfactory photochromic properties, moldability and adhesion. The photochromic laminate of Comparative Example 3 was unsatisfactory in terms of moldability and adhesion though it had satisfactory photochromic properties and surface hardness. Further, the photochromic laminate of Comparative Example 4 was unsatisfactory in terms of moldability though it was satisfactory in terms of photochromic properties, surface hardness and adhesion.

### Effect of the Invention

A laminate having a photochromic layer which exhibits excellent photochromic properties such as color optical density and fading speed and has high moldability and excellent surface hardness can be manufactured by using the photochromic coating composition of the present invention as shown in Examples which have been described above.

The development of the above photochromic properties is due to use of a polyrotaxane in combination with a photochromic compound. The inventors of the present invention consider the reason for this as follows.

That is, since the cyclic molecules of the polyrotaxane can slide over the axial molecule, a space is formed around the cyclic molecules and causes the reversible structural change of the photochromic compound swiftly with the result that the fading speed and the color optical density are improved. Further, the reversible structural change of the photochromic compound existent near the side chains having high flexibility can be caused more swiftly by introducing the cyclic molecules into which the side chains have been introduced.

Since color optical density and fading speed can be improved by the effect of the above polyrotaxane, a photochromic layer can be formed without impairing the crosslinking density of a urethane resin component and hydrogen bonding property. Therefore, excellent surface hardness is provided while excellent photochromic properties are retained.

Moreover, since the cyclic molecules of the polyrotaxane can slide over the axial molecule, stress caused by polymerization shrinkage which occurs when the photochromic layer is formed is mitigated, thereby providing high moldability without producing a crack in the photochromic layer and strain the obtained plastic lens.

## Claims

1. A method of producing a laminate, comprising the steps of:
coating a photochromic coating composition on an optical substrate by spin coating; and
leaving and curing the coated film of the photochromic coating composition on the optical substrate at a temperature range of 20 to 150°C to form a photochromic layer,
wherein the photochromic coating composition comprises (A) a polyrotaxane having a composite molecular structure formed by an axial molecule and a plurality of cyclic molecules clathrating the axial molecule, (B) a photochromic compound and (C2) a polyurethane resin precursor, and
wherein the polyurethane resin precursor (C2) includes (C2-1) a polyiso(thio)cyanate compound having at least two isocyanate groups and/or isothiocyanate groups in one molecule, (C2-2) a poly(thi)ol compound having at least two hydroxyl groups and/or thiol groups in one molecule and (C2-3) a mono(thi)ol compound having one hydroxyl group or thiol group in one molecule.

2. The method of producing a laminate according to claim 1, wherein a side chain having at least one polymerizable functional group selected from OH group, SH group, NH₂ group, NCO group and NCS group is introduced into at least one of the rings of the cyclic molecules of the polyrotaxane (A).

3. The method of producing a laminate according to claim 1 or 2, wherein the photochromic coating composition comprises 0.5 to 50 parts by mass of the polyrotaxane (A), 20 to 74 parts by mass of the polyiso(thio)cyanate compound (C2-1), 20 to 74 parts by mass of the poly(thi)ol compound (C2-2) and 2 to 40 parts by mass of the mono(thi)ol compound (C2-3) based on 100 parts by mass of the total of the polyrotaxane (A), the polyiso(thio)cyanate compound (C2-1), the poly(thi)ol compound (C2-2) and the mono(thi)ol compound (C2-3).

4. The method of producing a laminate according to any one of claims 1 to 3, wherein the thickness of the photochromic layer is 10 to 100 µm.

## Patentansprüche

1. Verfahren zur Herstellung eines Laminats, umfassend die folgenden Stufen:
das Aufschichten einer photochromen Beschichtungszusammensetzung auf ein optisches Substrat durch Schleuderbeschichten; und
das Stehenlassen und Aushärten des aufgeschichteten Films der photochromen Beschichtungszusammensetzung auf dem optischen Substrat bei einer Temperatur im Bereich von 20 bis 150°C, um eine photochrome Schicht zu bilden,
wobei die photochrome Beschichtungszusammensetzung umfasst: (A) ein Polyrotaxan mit einer molekularen Verbundstruktur, gebildet durch ein axiales Molekül und eine Mehrzahl von zyklischen Molekülen, die mit dem axialen Molekül ein Clathrat bilden, (B) eine photochrome Verbindung und (C2) einen Polyurethanharz-Vorläufer, und
wobei der Polyurethanharz-Vorläufer (C2) umfasst:
(C2-1) eine Polyiso(thio)cyanat-Verbindung mit mindestens zwei Isocyanat-Gruppen und/oder Isothiocyanat-Gruppen in einem Molekül, (C2-2) eine Poly(thi)ol-Verbindung mit mindestens zwei HydroxylGruppen und/oder Thiol-Gruppen in einem Molekül und
(C2-3) eine Mono(thi)ol-Verbindung mit einer HydroxylGruppe und/oder Thiol-Gruppe in einem Molekül.

2. Verfahren zur Herstellung eines Laminats nach Anspruch 1, wobei eine Seitenkette mit mindestens einer polymerisierbaren funktionellen Gruppe, ausgewählt aus OH-Gruppe, SH-Gruppe, NH₂-Gruppe, NCO-Gruppe und NCS-Gruppe, eingeführt ist in mindestens einen der Ringe der zyklischen Moleküle des Polyrotaxans (A).

3. Verfahren zur Herstellung eines Laminat nach Anspruch 1 oder 2, wobei die photochrome Beschichtungszusammensetzung umfasst: 0,5 bis 50 Masseteile des Polyrotaxans (A), 20 bis 74 Masseteile der Polyiso(thio)cyanat-Verbindung (C2-1), 20 bis 74 Masseteile der Poly(thi)ol-Verbindung (C2-2) und 2 bis 40 Masseteile der Mono(thi)ol-Verbindung (C2-3), bezogen auf 100 Masseteile der Gesamtmenge des Polyrotaxans (A), der Polyiso(thio)cyanat-Verbindung (C2-1), der Poly(thi)ol-Verbindung (C2-2) und der Mono(thi)ol-Verbindung (C2-3).

4. Verfahren zur Herstellung eines Laminats nach einem der Ansprüche 1 bis 3, wobei die Dicke der photochromen Schicht 10 bis 100 µm beträgt.

## Revendications

1. Procédé de production d'un stratifié, comprenant les étapes consistant à :
revêtir d'une composition de revêtement photochrome un substrat optique par dépôt à la tournette ; et
laisser et faire durcir le film revêtu de la composition de revêtement photochrome sur le substrat optique à une plage de températures de 20 à 150 °C pour former une couche photochrome,
dans lequel la composition de revêtement photochrome comprend (A) un polyrotaxane présentant une structure moléculaire composite formée par une molécule axiale et une pluralité de molécules cycliques de clathration de la molécule axiale, (B) un composé photochrome et (C2) un précurseur de résine de polyuréthanne, et
dans lequel le précurseur de résine de polyuréthanne (C2) inclut (C2-1) un composé polyiso(thio)cyanate présentant au moins deux groupes isocyanate et/ou groupes isothiocyanate dans une molécule, (C2-2) un composé poly(thi)ol présentant au moins deux groupes hydroxyle et/ou groupes thiol dans une molécule et (C2-3) un composé mono(thi)ol présentant un groupe hydroxyle ou groupe thiol dans une molécule.

2. Procédé de production d'un stratifié selon la revendication 1, dans lequel une chaîne latérale présentant au moins un groupe fonctionnel polymérisable sélectionné parmi un groupe OH, un groupe SH, un groupe NH₂, un groupe NCO et un groupe NCS est introduite dans au moins un des cycles des molécules cycliques du polyrotaxane (A).

3. Procédé de production d'un stratifié selon la revendication 1 ou 2, dans lequel la composition de revêtement photochrome comprend 0,5 à 50 parties en masse du polyrotaxane (A), 20 à 74 parties en masse du composé polyiso(thio)cyanate (C2-1), 20 à 74 parties en masse du composé poly(thi)ol (C2-2) et 2 à 40 parties en masse du composé mono(thi)ol (C2-3) sur la base de 100 parties en masse du total du polyrotaxane (A), du composé polyiso(thio)cyanate (C2-1), du composé poly(thi)ol (C2-2) et du composé mono(thi)ol (C2-3).

4. Procédé de production d'un stratifié selon l'une quelconque des revendications 1 à 3, dans lequel l'épaisseur de la couche photochrome est de 10 à 100 µm.
